# EUROPEAN PATENT APPLICATION

(11) **EP 2 080 506 A1**
(43) Date of publication of application: **22.07.2009**
(21) Application number: 06828334.0
(22) Date of filing: 15.12.2006
(51) Int. Cl.: A61K 8/97, A61Q 19/00, A61Q 19/08

(54) **COSMETIC COMPOSITION AND PREPARATION METHOD AND USE THEREOF**

(30) Priority: 17.10.2006 CN 200610117216
(71) Applicant: Wang, Hailong, Shanghai 200433 (CN); Fang, Zongren, Shanghai 200433 (CN); Zhang, Xiaoping, Shanghai 200433 (CN); Venne, Dominic, Shanghai 200433 (CN); Stumpo, David Martin, Shanghai 200433 (CN)
(72) Inventor: Wang, Hailong, Shanghai 200433 (CN); Fang, Zongren, Shanghai 200433 (CN); Zhang, Xiaoping, Shanghai 200433 (CN); Venne, Dominic, Shanghai 200433 (CN); Stumpo, David Martin, Shanghai 200433 (CN)
(74) Representative: Cabinet Plasseraud
(86) International application number: PCT/CN2006/003420
(87) International publication number: WO 2008/046258

(57) **Abstract**

A skin-whitening, freckle-removing cosmetic composition, comprising an effective amount of extract from Cistanche deserticola and other useful adjuvants.

## Description

### CROSS REFERENCE TO RELATED PATENT APPLICATION

This patent application is a PCT application No. PCT/CN2006/003420 with International Filing Date of December 15, 2006 entering U.S.A. national stage, which claims the priority of Chinese patent application No. 200610117216.1 filed on October 17, 2006, the above-mentioned two applications are enclosed herein by reference.

### FIELD OF THE INVENTION

The present invention relates generally to a cosmetic composition comprising an extract that is derived from a plant, a process for preparing same and a use thereof. More particularly, the invention relates to a dermatologic and cosmetic preparation comprising an extract derived from a plant, which possesses skin-whitening and freckle-removing actions and makes the skin more youthful looking. The present invention also relates to a process for preparing said preparation.

### BACKGROUND OF THE INVENTION

With the elevation of living standard, women of all ages pay more and more attention to the skin health, and maintaining a youthful looking skin becomes an on-going need for women. To improve the youthful appearance of skin generally includes the improvement of overall skin tone, reduction in pigmentation and spots, and increases in suppleness and softness. Genus cistanche is a traditional Chinese medicine of kidney-invigoration, having pneuma-tonifing and antidotal actions. Its history as both a drug and a food for the double purpose is over two thousand years.

Japan scholars have recently found that phenylethanoid glycosides, as active components obtained from plants, are of more potent tyrosinase-inhibiting activity and chromatogenesis-inhibiting activity. It has been shown, in vitro, that phenylethanoid glycosides possess an active promotion on cytolergy of M cells to form a collagen fiber network (Tanimoto S, et al., Yakugaku Zasshi. 2006 Mar; 126(3):173-7. Sudo H et all. Chem Pharm Bull (Tokyo). 1999 Sep; 47(9):1341-3.Related).

It has been found that the extract from Genus cistanche contains phenylethanoid glycosides. Among all species of the genus cistanche, Cistanche tubulosa (Schenk.) Wight produced in southern Xinjiang, China, contains the greatest amount of phenylethanoid glycosides, thus is expectable to be used in skin care preparations to improve skin health and produce agerasia and facial actions.

### SUMMARY OF THE INVENTION

The primary object of the present invention is to provide a cosmetic composition comprising an extract obtained from Genus cistanche. It is another object of the present invention to provide a process for preparing an extract which is rich in phenylethanoid glycosides from Cistanche tubulosa (Schenk.) wight. It is still another object of the present invention to provide the application of the cosmetic composition containing such extract of Cistanche tubulosa (Schenk.) Wight.

For the above objects, the solutions of the present invention are as follows: A cosmetic composition comprising an effective amount of extract of Cistanche tubulosa (Schenk.) Wight and carrier(s) acceptable in cosmetic.

The extract of Cistanche tubulosa, especially, the extract from Cistanche tubulosa (Schenk.) Wight, is rich in phenylethanoid glycosides. Preferrablely, the extract comprises at least 2% of acteoside by weight, and at least 4% of echinaoside by weight, based on the total weight of the extract. As main active ingredients, the higher the amounts of acteoside and echinaoside are, the better the effect is. However, in view of various factors such as the commercial cost , it is impossible to make unlimited refines to raise the contents of these two active ingredients. Therefore, once the content of acteoside reaches 2-80% by weight and the content of echinaoside reaches 4-80% by weight, the effect is desirable. Most preferrablely, the weight radio of acteoside to echinaoside is over 2:1.

Besides that the extract from Cistanche tubulosa (Schenk.) wight is rich in phenylethanoid glycosides, the extract also comprises acteoside isomer, 2'acetylacteoside and tubuloside A, B, C, each being in an amount less than 4% by weight based on the total weight of the extract. The contents of acteoside and echinaoside are measured by HPLC, a method of content determination recorded in page 90 of China Pharmacopeia I (Ed. 2005).

Phenylethanoid glycosides have general formula of:

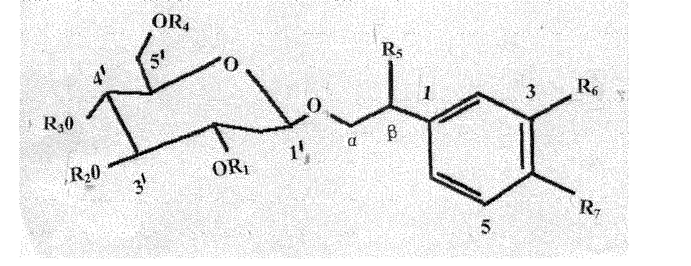

| | | | | | | | |
|---|---|---|---|---|---|---|---|
| Ingredient | R1 | R2 | R3 | R4 | R5 | R6 | R7 |
| Acteoside | H | RHa | Cf | H | H | OH | OH |
| Echinacoside | H | RHa | Cf | Glc | H | OH | OH |
| 2'acetylacteoside | Ac | RHa | Cf | H | H | OH | OH |
| acteoside isomer | H | RHa | H | Cf | H | OH | OH |
| tubuloside A | H | RHa | Cf | Glc | H | H | OH |
| tubuloside B | H | RHa | Cm | Glc | H | OH | OH |
| tubuloside C | H | RHa | Cm | Glc | H | OH | OH |

The carrier(s) used in the cosmetic compositions of the present invention is/are dermatologically acceptable carrier(s) that is/are suitable for topical application to the keratinous tissue, and compatible with the active ingredients of the present invention and any other additives, without any concern of untoward safety or toxicity to the skin. A safe and effective amount of carrier(s) is from about 10% to about 99.99%, preferably from about 20% to about 99.9%, more preferably from about 40% to about 98%, based on the total weight of the composition.

The carrier can be in a wide variety of forms. For example, emulsion carriers, including, but not limited to, oil-in-water, water-in-oil, water-in-oil-in-water (W/O/W), and oil-in-water-in-silicone (W/O/S) emulsions, are useful herein.

Preferred carriers contain an emulsion such as oil-in-water emulsions, water-in-oil emulsions, and water-in-silicone emulsions. As will be understood by the skilled artisan, a given component will distribute primarily into either the water or oil/silicone phase, depending on the water solubility and dispersibility of the component in the composition.

Emulsions according to the present invention generally contain Lipids and oils which may be derived from animals, plants or petroleum and may be natural or synthetic. Preferred emulsions also contain a humectant, such as glycerin or propylene glycol or butylenes glycol or sorbitol. Emulsions will preferably further contain from about 0.01% to about 10%, more preferably from about 0.1% to about 5%, of an emulsifier, based on the weight of the carrier. Emulsifiers may be nonionic, anionic or cationic. Suitable emulsifiers are disclosed in, for example, U.S. Pat. No. 3,755,560, and McCutcheon's Detergents and Emulsifiers, North American Edition, pages 317-324 (1986).

The emulsion may also contain an anti-foaming agent to minimize foaming. Anti-foaming agents include high molecular weight silicones and other materials well known in the art for such use.

Water-in-silicone emulsions contain a continuous silicone phase and a dispersed aqueous phase.

The organopolysiloxane oil for use in the emulsion may be volatile, non-volatile, or a mixture of volatile and non-volatile silicones. The term "nonvolatile" as used in this context refers to those silicones that are liquid under ambient conditions and have a flash point (under one atmospheric of pressure) of greater than 100 degree Centigrade. The term "volatile" as used in this context refers to all other silicone oils. Suitable organopolysiloxanes can be selected from a wide variety of silicones spanning a broad range of volatilities and viscosities. Examples of suitable organopolysiloxane oils include polyalkylsiloxanes, cyclic polyalkylsiloxanes and polyalkylarylsiloxanes.

Polyalkylsiloxanes useful in the composition herein include polyalkylsiloxanes with viscosities of from about 0.5 to about 1,000,000 centistokes at 25°C. Such polyalkylsiloxanes can be represented by the general formula R₃ SiO[R₂SiO]ₓ SiR₃ wherein R is an alkyl group having from one to about 30 carbon atoms (preferably R is methyl or ethyl, more preferably ethyl), and x is an integer from 0 to about 10,000, chosen to achieve the desired molecular weight. Commercially available polyalkylsiloxanes include the polydimethylsiloxanes, examples of which include the Vicasil.RTM. series sold by General Electric Company and the Dow Coming.RTM. 200 series sold by Dow Coming Corporation. Specific examples of suitable polydimethylsiloxanes include Dow Coming.RTM. 200 fluid having a viscosity of 0.65 centistokes and a boiling point of 100°C., Dow Coming.RTM. 225 fluid having a viscosity of 10 centistokes and a boiling point greater than 200°C, and Dow Coming.RTM. 200 fluids having viscosities of 50, 350, and 12,500 centistokes, respectively, and boiling points greater than 200°C. Suitable dimethicones include those represented by the formula (CH₃)₃SiO[(CH₃)₂SiO]ₓ[CH₃RSiO]_{y}Si(CH₃)₃ wherein R is a straight or branched chain alkyl having from two to about 30 carbon atoms and x and y are each integers of 1 to about 10,000,000 selected to achieve the desired molecular weight. Examples of these alkyl-substituted dimethicones include cetyl dimethicone and lauryl dimethicone.

Cyclic polyalkylsiloxanes suitable for use in the composition as carrier(s) include those represented by the general chemical formula [SiR₂--O]ₙ wherein R is an alkyl group (preferably R is methyl or ethyl, more preferably methyl) and n is an integer from about 3 to about 8, more preferably n is an integer from about 3 to about 7, and still more preferably n is an integer from about 4 to about 6. When R is methyl, these materials are typically referred to as cyclomethicones. Commercially available cyclomethicones include Dow Corning. RTM. 244 fluid having a viscosity of 2.5 centistokes, and a boiling point of 172°C, which primarily contains the cyclomethicone tetramer (i.e. n=4), Dow Coming.RTM. 344 fluid having a viscosity of 2.5 centistokes and a boiling point of 178°C, which primarily contains the cyclomethicone pentamer (i.e. n=5), Dow Coming.RTM. 245 fluid having a viscosity of 4.2 centistokes and a boiling point of 205°C, which primarily contains a mixture of the cyclomethicone tetramer and pentamer (i.e. n=4 and 5), and Dow Coming.RTM. 345 fluid having a viscosity of 4.5 centistokes and a boiling point of 217°C, which primarily contains a mixture of the cyclomethicone tetramer, pentamer, and hexamer (i.e. n=4, 5, and 6).

Also useful are materials such as trimethylsiloxysilicate, which is a polymeric material corresponding to the general chemical formula [(CH₂)₃ SiO_{1/2} ]ₓ [SiO₂ ]_{y}, wherein x is an integer from about 1 to about 500 and y is an integer from about 1 to about 500. A commercially available trimethylsiloxysilicate is sold as a mixture with dimethicone and Dow Coming.RTM. 593 fluid.

Dimethiconols are also suitable for use in the cosmetic composition. These compounds can be represented by the general chemical formulas R₃ SiO[R₂SiO]ₓSiR₂OH and HOR₂SiO[R₂SiO]ₓSiR₂OH wherein R is an alkyl group (preferably R is methyl or ethyl, more preferably methyl) and x is an integer from 0 to about 500, chosen to achieve the desired molecular weight. Commercially available dimethiconols are typically sold as mixtures with dimethicone and cyclomethicone (e.g. Dow Coming.RTM. 1401, 1402, and 1403 fluids).

Polyalkylaryl siloxanes are also suitable for use in the cosmetic composition. Polymethylphenyl siloxanes having viscosities from about 15 to about 65 centistokes at 25°C are especially useful.

Preferred for use herein are organopolysiloxanes selected from any one of polyalkylsiloxanes, alkyl substituted dimethicones, cyclomethicones, trimethylsiloxysilicates, dimethiconols, polyalkylaryl siloxanes, and any combination thereof. Preferred among the polyalkylsiloxanes are dimethicones.

The cosmetic composition may additionally contain one or more non-silicone oils. Suitable non-silicone oils have a melting point of about 25°C or less. Examples of such non-silicone oils are those well known in the chemical arts in the form of water-in-oil emulsions, e.g., mineral oil, vegetable oils, synthetic lipids, and semisynthetic lipids, etc.

Dispersed Aqueous Phase when water in silicone emulsion is used.

The cosmetic compositions of the present invention contain from about 10% to about 90%, more preferably from about 20% to about 85%, and still more preferably from about 30% to about 80% of a dispersed aqueous phase. The dispersed phase is known as the internal phase or discontinuous phase. The dispersed aqueous phase is a dispersion of small aqueous particles or droplets suspended in and surrounded by the continuous silicone phase described hereinbefore.

The aqueous phase can be water, or a combination of water and one or more water soluble or dispersible ingredients. Nonlimiting examples of such ingredients include thickeners, acids, bases, salts, chelants, gums, water-soluble or dispersible alcohols and polyols, buffers, preservatives, sunscreening agents, colorings, and the like.

The cosmetic compositions of the present invention will typically contain from about 25% to about 90%, preferably from about 40% to about 80%, more preferably from about 60% to about 80%, water in the dispersed aqueous phase by weight of the composition.

### Emulsifier for Dispersing the Aqueous Phase

The water-in-silicone emulsions of the present invention preferably contain an emulsifier. In a preferred embodiment, the composition contains from about 0.1% to about 10% emulsifier, more preferably from about 0.5% to about 7.5%, still more preferably from about 1% to about 5%, emulsifier by weight of the composition. The emulsifier helps disperse and suspend the aqueous phase within the continuous silicone phase.

A wide variety of emulsifying agents can be employed herein to form the water-in-silicone emulsion. Known or conventional emulsifying agents can be used in the present invention, provided that the selected emulsifying agent is chemically and physically compatible with components of the composition of the present invention, and provides the desired dispersion characteristics. Suitable emulsifiers include silicone emulsifiers, non-silicon-containing emulsifiers, and mixtures thereof, known by those skilled in the art for use in topical personal care products. Preferably these emulsifiers have an HLB value (hydrophile-lipophile balance value) of or less than about 14, more preferably from about 2 to about 14, and still more preferably from about 4 to about 14. Emulsifiers having an HLB value outside of these ranges can be used in combination with other emulsifiers so to adjust the HLB value to fall within these ranges, thus to achieve an equal effect.

Silicone emulsifiers are preferred. A wide variety of silicone emulsifiers are useful herein. These silicone emulsifiers are typically organically modified organopolysiloxanes, also known to those skilled in the art as silicone surfactants. Useful silicone emulsifiers include dimethicone copolyols. These materials are polydimethyl siloxanes which have been modified to include polyether side chains such as polyethylene oxide chains, polypropylene oxide chains, mixtures of these chains, and polyether chains containing moieties derived from both ethylene oxide and propylene oxide. Other examples include alkyl-modified dimethicone copolyols, i.e., compounds which contain C₂-C₃₀ pendant side chains. Still other useful dimethicone copolyols include materials having various cationic, anionic, amphoteric, and zwitterionic pendant moieties.

Also useful herein, although not strictly classified as dimethicone copolyols, are silicone surfactants.

Nonlimiting examples of dimethicone copolyols and other silicone surfactants useful as emulsifiers herein include polydimethylsiloxane polyether copolymers with pendant polyethylene oxide sidechains, polydimethylsiloxane polyether copolymers with pendant polypropylene oxide sidechains, polydimethylsiloxane polyether copolymers with pendant mixed polyethylene oxide and polypropylene oxide sidechains, polydimethylsiloxane polyether copolymers with pendant mixed poly(ethylene)(propylene)oxide sidechains, polydimethylsiloxane polyether copolymers with pendant organobetaine sidechains, polydimethylsiloxane polyether copolymers with pendant carboxylate sidechains, polydimethylsiloxane polyether copolymers with pendant quaternary ammonium sidechains; and also further modifications of the preceding copolymers containing pendant C₂-C₃₀ straight, branched, or cyclic alkyl moieties. Examples of commercially available dimethicone copolyols useful herein sold by Dow Coming Corporation are Dow Coming.RTM. 190, 193, Q2-5220, 2501 Wax, 2-5324 fluid, and 3225C (this later material being sold as a mixture with cyclomethicone). Cetyl dimethicone copolyol is commercially available as a mixture with polyglyceryl-4 isostearate (and) hexyl laurate and is sold under the tradename ABIL.RTM. WE-09 (available from Goldschmidt). Cetyl dimethicone copolyol is also commercially available as a mixture with hexyl laurate (and) polyglyceryl-3 oleate (and) cetyl dimethicone and is sold under the tradename ABIL.RTM. WS-08 (also available from Goldschmidt). Other nonlimiting examples of dimethicone copolyols also include lauryl dimethicone copolyol, dimethicone copolyol acetate, diemethicone copolyol adipate, dimethicone copolyolamine, dimethicone copolyol behenate, dimethicone copolyol butyl ether, dimethicone copolyol hydroxy stearate, dimethicone copolyol isostearate, dimethicone copolyol laurate, dimethicone copolyol methyl ether, dimethicone copolyol phosphate, and dimethicone copolyol stearate. See International Cosmetic Ingredient Dictionary, Fifth Edition, 1993.

Dimethicone copolyol emulsifiers useful herein are described, for example, in U.S. Pat. No. 4,960,764, to Figueroa, Jr. et al., issued Oct. 2, 1990; European Patent No. EP 330,369, to SanoGueira, published Aug. 30, 1989; G. H. Dahms, et al., "New Formulation Possibilities Offered by Silicone Copolyols," Cosmetics & Toiletries, vol. 110, pp. 91-100, March 1995; M. E. Carlotti et al., "Optimization of W/O-S Emulsions And Study Of The Quantitative Relationships Between Ester Structure And Emulsion Properties," J. Dispersion Science And Technology, 13(3), 315-336 (1992); P. Hameyer, "Comparative Technological Investigations of Organic and Organosilicone Emulsifiers in Cosmetic Water-in-Oil Emulsion Preparations," HAPPI 28(4), pp. 88-128 (1991); J. Smid-Korbar et al., "Efficiency and usability of silicone surfactants in emulsions," Provisional Communication, International Journal of Cosmetic Science, 12, 135-139 (1990); and D. G. Krzysik et al., "A New Silicone Emulsifier For Water-in-Oil Systems," Drug and Cosmetic Industry, vol. 146(4) pp. 28-81 (April 1990).

Among the non-silicone-containing emulsifiers useful herein are various non-ionic and anionic emulsifying agents such as sugar esters and polyesters, alkoxylated sugar esters and polyesters, C₁-C₃₀ fatty acid esters of C₁-C₃₀ fatty alcohols, alkoxylated derivatives of C₁-C₃₀ fatty acid esters of C₁-C₃₀ fatty alcohols, alkoxylated ethers of C₁-C₃₀ fatty alcohols, polyglyceryl esters of C₁-C₃₀ fatty acids, C₁-C₃₀ esters of polyols, C₁-C₃₀ ethers of polyols, alkyl phosphates, polyoxyalkylene fatty ether phosphates, fatty acid amides, acyl lactylates, soaps, and mixtures thereof. Other suitable emulsifiers are described, for example, in McCutcheon's, Detergents and Emulsifiers, North American Edition (1986), published by Allured Publishing Corporation; U.S. Pat. No. 5,011,681 to Ciotti et al., issued Apr. 30, 1991; U.S. Pat. No. 4,421,769 to Dixon et al., issued Dec. 20, 1983; and U.S. Pat. No. 3,755,560 to Dickert et al., issued Aug. 28, 1973.

Nonlimiting examples of these non-silicon-containing emulsifiers include: polyethylene glycol 20 sorbitan monolaurate (Polysorbate 20), polyethylene glycol 5 soya sterol, Steareth-20, Ceteareth-20, PPG-2 methyl glucose ether distearate, Ceteth-10, Polysorbate 80, cetyl phosphate, potassium cetyl phosphate, diethanolamine cetyl phosphate, Polysorbate 60, glyceryl stearate, PEG-100 stearate, polyoxyethylene 20 sorbitan trioleate (Polysorbate 85), sorbitan monolaurate, polyoxyethylene 4 lauryl ether sodium stearate, polyglyceryl-4 isostearate, hexyl laurate, steareth-20, ceteareth-20, PPG-2 methyl glucose ether distearate, ceteth-10, diethanolamine cetyl phosphate, glyceryl stearate, PEG-100 stearate, and mixtures thereof.

### Oil-in-Water Emulsions

Other preferred carriers include oil-in-water emulsions, having a continuous aqueous phase and a water-insoluble phase ("oil phase") dispersed therein. Examples of suitable oil-in-water emulsion are described in U.S. Pat. No. 5,073,371, to Turner, D. J. et al., issued Dec. 17, 1991, and U.S. Pat. No. 5,073,372, to Turner, D. J. et al., issued Dec. 17, 1991. A preferred oil-in-water emulsion, containing a structuring agent, hydrophilic surfactant and water, is described in detail hereinafter.

### (1) Structuring Agent

A preferred oil-in-water emulsion contains a structuring agent to assist in the formation of a liquid crystalline gel network structure. Without being limited by theory, it is believed that the structuring agent assists in providing rheological characteristics to the composition which contribute to the stability of the composition. The structuring agent may also function as an emulsifier or surfactant. The emulsion according to the invention contains from about 0.5% to about 20%, more preferably from about 1% to about 10%, even more preferably from about 1% to about 5%, by weight of the composition, of a structuring agent.

The preferred structuring agents of the present invention include stearic acid, palmitic acid, stearyl alcohol, cetyl alcohol, behenyl alcohol, stearic acid, palmitic acid, the polyethylene glycol ether of stearyl alcohol having an average of about 1 to about 21 ethylene oxide units, the polyethylene glycol ether of cetyl alcohol having an average of about 1 to about 7 ethylene oxide units, and mixtures thereof. Other structuring agents used in the present invention are selected from stearyl alcohol, cetyl alcohol, behenyl alcohol, the polyethylene glycol ether of stearyl alcohol having an average of about 2 ethylene oxide units (steareth-2), the polyethylene glycol ether of stearyl alcohol having an average of about 21 ethylene oxide units (steareth-21), and mixtures thereof. The structuring agents can also be one which is selected from stearic acid, palmitic acid, stearyl alcohol, cetyl alcohol, behenyl alcohol, steareth-2, steareth-21, and mixtures thereof.

### (2) Hydrophilic Surfactant

The preferred oil-in-water emulsions contain from about 0.05% to about 10%, preferably from about 1% to about 6%, and more preferably from about 1% to about 5% (percentages by weight of the total emulsion) of at least one hydrophilic surfactant which can disperse the hydrophobic materials in the water phase. The surfactant, at a minimum, must be hydrophilic enough to disperse in water.

Preferred hydrophilic surfactants are selected from nonionic surfactants. Among the nonionic surfactants that are useful herein are those that can be broadly defined as condensation products of long chain alcohols, e.g. C₈₋₃₀ alcohols, with sugar or starch polymers, i.e., glycosides. These compounds can be represented by the formula (S)ₙ --O--R wherein S is a sugar moiety such as glucose, fructose, mannose, and galactose; n is an integer of from about 1 to about 1000, and R is a C₈₋₃₀ alkyl group. Examples of long chain alcohols from which the alkyl group can be derived include decyl alcohol, cetyl alcohol, stearyl alcohol, lauryl alcohol, myristyl alcohol, oleyl alcohol, and the like. Preferred examples of these surfactants include those wherein S is a glucose moiety, R is a C₈₋₂₀ alkyl group, and n is an integer of from about 1 to about 9. Commercially available examples of these surfactants include decyl polyglucoside (available as APG 325 CS from Henkel) and lauryl polyglucoside (available as APG 600 CS and 625 CS from Henkel).

Other useful nonionic surfactants include the condensation products of alkylene oxides with fatty acids (i.e. alkylene oxide esters of fatty acids). These materials have the general formula RCO(X)ₙOH wherein R is a C₁₀₋₃₀ alkyl group, X is --OCH₂CH₂-- (i.e. derived from ethylene glycol or oxide) or --OCH₂CHCH₃-- (i.e. derived from propylene glycol or oxide), and n is an integer from about 6 to about 200. Other nonionic surfactants are the condensation products of alkylene oxides with 2 moles of fatty acids (i.e. alkylene oxide diesters of fatty acids). These materials have the general formula RCO(X)ₙOOCR wherein R is a C₁₀₋₃₀ alkyl group, X is --OCH₂CH₂-- (i.e. derived from ethylene glycol or oxide) or --OCH₂CHCH₃-- (i.e. derived from propylene glycol or oxide), and n is an integer from about 6 to about 100. Other nonionic surfactants are the condensation products of alkylene oxides with fatty alcohols (i.e. alkylene oxide ethers of fatty alcohols). These materials have the general formula R(X).sub.n OR' wherein R is a C10-30 alkyl group, X is --OCH₂CH₂-- (i.e. derived from ethylene glycol or oxide) or --OCH₂CHCH₃-- (i.e. derived from propylene glycol or oxide), and n is an integer from about 6 to about 100 and R' is H or a C₁₀₋₃₀ alkyl group. Still other nonionic surfactants are the condensation products of alkylene oxides with both fatty acids and fatty alcohols [i.e. wherein the polyalkylene oxide portion is esterified on one end with a fatty acid and etherified (i.e. connected via an ether linkage) on the other end with a fatty alcohol]. These materials have the general formula RCO(X)ₙOR' wherein R and R' are C₁₀₋₃₀ alkyl groups, X is --OCH₂ CH₂ (i.e. derived from ethylene glycol or oxide) or --OCH₂CHCH₃-(derived from propylene glycol or oxide), and n is an integer from about 6 to about 100. Nonlimiting examples of these alkylene oxide derived nonionic surfactants include ceteth-6, ceteth-10, ceteth-12, ceteareth-6, ceteareth-10, ceteareth-12, steareth-6, steareth-10, steareth-12, steareth-21, PEG-6 stearate, PEG-10 stearate, PEG-100 stearate, PEG-12 stearate, PEG-20 glyceryl stearate, PEG-80 glyceryl tallowate, PEG-10 glyceryl stearate, PEG-30 glyceryl cocoate, PEG-80 glyceryl cocoate, PEG-200 glyceryl tallowate, PEG-8 dilaurate, PEG-10 distearate, and mixtures thereof.

Preferred among the nonionic surfactants are those selected from the group consisting of steareth-21, ceteareth-20, ceteareth-12, sucrose cocoate, steareth-100, PEG-100 stearate, and mixtures thereof.

Other nonionic surfactants suitable for use herein include sugar esters and polyesters, alkoxylated sugar esters and polyesters, C₁-C₃₀ fatty acid esters of C₁-C₃₀ fatty alcohols, alkoxylated derivatives of C₁-C₃₀ fatty acid esters of C₁-C₃₀ fatty alcohols, alkoxylated ethers of C₁-C₃₀ fatty alcohols, polyglyceryl esters of C₁-C₃₀ fatty acids, C₁-C₃₀esters of polyols, C₁-C₃₀ ethers of polyols, alkyl phosphates, polyoxyalkylene fatty ether phosphates, fatty acid amides, acyl lactylates, and mixtures thereof. Nonlimiting examples of these emulsifiers include: polyethylene glycol 20 sorbitan monolaurate (Polysorbate 20), polyethylene glycol 5 soya sterol, Steareth-20, Ceteareth-20, PPG-2 methyl glucose ether distearate, Ceteth-10, Polysorbate 80, cetyl phosphate, potassium cetyl phosphate, diethanolamine cetyl phosphate, Polysorbate 60, glyceryl stearate, polyoxyethylene 20 sorbitan trioleate (Polysorbate 85), sorbitan monolaurate, polyoxyethylene 4 lauryl ether sodium stearate, polyglyceryl-4 isostearate, hexyl laurate, PPG-2 methyl glucose ether distearate, PEG-100 stearate, and mixtures thereof.

Another emulsifier useful herein are fatty acid ester blends based on a mixture of sorbitan or sorbitol fatty acid ester and sucrose fatty acid ester, the fatty acid in each instance being preferably C₈ -C₂₄, more preferably C₁₀ -C₂₀. The preferred fatty acid ester emulsifier is a blend of sorbitan or sorbitol C₁₆ -C₂₀ fatty acid ester with sucrose C₁₀ -C₁₆ fatty acid ester, especially sorbitan stearate and sucrose cocoate. This is commercially available from ICI under the trade name Arlatone 2121.

Other suitable surfactants useful herein include a wide variety of cationic, anionic, zwitterionic, and amphoteric surfactants such as are known in the art. See, e.g., McCutcheon's, Detergents and Emulsifiers, North American Edition (1986), published by Allured Publishing Corporation; U.S. Pat. No. 5,011,681 to Ciotti et al., issued Apr. 30, 1991; U.S. Pat. No. 4,421,769 to Dixon et al., issued Dec. 20, 1983; and U.S. Pat. No. 3,755,560 to Dickert et al., issued Aug. 28, 1973; these four references are incorporated herein by reference in their entirety. The hydrophilic surfactants useful herein can contain a single surfactant, or any combination of suitable surfactants. The exact surfactant (or surfactants) chosen will depend upon the pH of the composition and the other components present.

Also useful herein are cationic surfactants, especially dialkyl quaternary ammonium compounds, examples of which are described in U.S. Pat. Nos. 5,151,209; 5,151,210; 5,120,532; 4,387,090; 3,155,591; 3,929,678; 3,959,461; McCutcheon's, Detergents & Emulsifiers, (North American edition 1979) M.C. Publishing Co.; and Schwartz, et al., Surface Active Agents, Their Chemistry and Technology, New York: Interscience Publishers, 1949; which descriptions are incorporated herein by reference.

Alternatively, other useful cationic emulsifiers include amino-amides. Nonlimiting examples of these cationic emulsifiers include stearamidopropyl PG-dimonium chloride phosphate, behenamidopropyl PG dimonium chloride, stearamidopropyl ethyldimonium ethosulfate, stearamidopropyl dimethyl(myristyl acetate)ammonium chloride, stearamidopropyl dimethyl cetearyl ammonium tosylate, stearamidopropyl dimethyl ammonium chloride, stearamidopropyl dimethyl ammonium lactate, and mixtures thereof. Especially preferred is behenamidopropyl PG dimonium chloride.

Nonlimiting examples of quaternary ammonium salt cationic surfactants include those selected from cetyl ammonium chloride, cetyl ammonium bromide, lauryl ammonium chloride, lauryl ammonium bromide, stearyl ammonium chloride, stearyl ammonium bromide, cetyl dimethyl ammonium chloride, cetyl dimethyl ammonium bromide, lauryl dimethyl ammonium chloride, lauryl dimethyl ammonium bromide, stearyl dimethyl ammonium chloride, stearyl dimethyl ammonium bromide, cetyl trimethyl ammonium chloride, cetyl trimethyl ammonium bromide, lauryl trimethyl ammonium chloride, lauryl trimethyl ammonium bromide, stearyl trimethyl ammonium chloride, stearyl trimethyl ammonium bromide, lauryl dimethyl ammonium chloride, stearyl dimethyl cetyl ditallow dimethyl ammonium chloride, dicetyl ammonium chloride, dicetyl ammonium bromide, dilauryl ammonium chloride, dilauryl ammonium bromide, distearyl ammonium chloride, distearyl ammonium bromide, dicetyl methyl ammonium chloride, dicetyl methyl ammonium bromide, dilauryl methyl ammonium chloride, dilauryl methyl ammonium bromide, distearyl methyl ammonium chloride, distearyl methyl ammonium bromide, and mixtures thereof. Additional quaternary ammonium salts include those wherein the C₁₂ to C₃₀ alkyl carbon chain is derived from a tallow fatty acid or from a coconut fatty acid. The term "tallow" refers to an alkyl group derived from tallow fatty acids (usually hydrogenated tallow fatty acids), which generally have mixtures of alkyl chains in the C₁₆ to C₁₈ range. The term "coconut" refers to an alkyl group derived from a coconut fatty acid, which generally have mixtures of alkyl chains in the C₁₂ to C₁₄ range. Examples of quaternary ammonium salts derived from these tallow and coconut sources include ditallow dimethyl ammonium chloride, ditallow dimethyl ammonium methyl sulfate, di(hydrogenated tallow)dimethyl ammonium chloride, di(hydrogenated tallow)dimethyl ammonium acetate, ditallow dipropyl ammonium phosphate, ditallow dimethyl ammonium nitrate, di(coconutalkyl)dimethyl ammonium chloride, di(coconutalkyl)dimethyl ammonium bromide, tallow ammonium chloride, coconut ammonium chloride, stearamidopropyl PG-dimonium chloride phosphate, stearamidopropyl ethyldimonium ethosulfate, stearamidopropyl dimethyl(myristyl acetate)ammonium chloride, stearamidopropyl dimethyl cetearyl ammonium tosylate, stearamidopropyl dimethyl ammonium chloride, stearamidopropyl dimethyl ammonium lactate, and mixtures thereof. An example of a quaternary ammonium compound having an alkyl group with an ester linkage is ditallowyl oxyethyl dimethyl ammonium chloride.

More preferred cationic surfactants are those selected from behenamidopropyl PG dimonium chloride, dilauryl dimethyl ammonium chloride, distearyl dimethyl ammonium chloride, dimyristyl dimethyl ammonium chloride, dipalmityl dimethyl ammonium chloride, distearyl dimethyl ammonium chloride, stearamidopropyl PG-dimonium chloride phosphate, stearamidopropyl ethyldiammonium ethosulfate, stearamidopropyl dimethyl(myristyl acetate)ammonium chloride, stearamidopropyl dimethyl cetearyl ammonium tosylate, stearamidopropyl dimethyl ammonium chloride, stearamidopropyl dimethyl ammonium lactate, and mixtures thereof.

Still more preferred cationic surfactants are those selected from behenamidopropyl PG dimonium chloride, dilauryl dimethyl ammonium chloride, distearyl dimethyl ammonium chloride, dimyristyl dimethyl ammonium chloride, dipalmityl dimethyl ammonium chloride, and mixtures thereof.

A preferred combination of cationic surfactant and structuring agent is behenamidopropyl PG dimonium chloride and/or behenyl alcohol, wherein the ratio is preferably optimized to maintain to enhance physical and chemical stability, especially when such a combination contains ionic and/or highly polar solvents. This combination is especially useful for delivery of sunscreening agents such as zinc oxide and octyl methoxycinnamate.

A wide variety of anionic surfactants are also useful herein. See, e.g., U.S. Pat. No. 3,929,678, to Laughlin et al., issued Dec. 30, 1975, which is incorporated herein by reference in its entirety. Nonlimiting examples of anionic surfactants include the alkoyl isethionates, and the alkyl and alkyl ether sulfates. The alkoyl isethionates typically have the formula RCO--OCH₂CH₂SO₃M wherein R is alkyl or alkenyl of from about 10 to about 30 carbon atoms, and M is a water-soluble cation such as ammonium, sodium, potassium and triethanolamine. Nonlimiting examples of these isethionates include those alkoyl isethionates selected from ammonium cocoyl isethionate, sodium cocoyl isethionate, sodium lauroyl isethionate, sodium stearoyl isethionate, and mixtures thereof.

The alkyl and alkyl ether sulfates typically have the respective formulae ROSO₃M and RO(C₂H₄O)ₓSO₃M, wherein R is alkyl or alkenyl of from about 10 to about 30 carbon atoms, x is an integer of from about 1 to about 10, and M is a water-soluble cation such as ammonium, sodium, potassium and triethanolamine.

Other anionic materials useful herein are soaps (i.e. alkali metal salts, e.g., sodium or potassium salts) of fatty acids, typically having from about 8 to about 24 carbon atoms, preferably from about 10 to about 20 carbon atoms. The fatty acids used in making the soaps can be obtained from natural sources such as, for instance, plant or animal-derived glycerides (e.g., palm oil, coconut oil, soybean oil, castor oil, tallow, lard, etc.) The fatty acids can also be synthetically prepared. Soaps are described in more detail in U.S. Pat. No. 4,557,853.

Amphoteric and zwitterionic surfactants are also useful herein. Examples of amphoteric and zwitterionic surfactants which can be used in the cosmetic compositions of the present invention are those which are broadly described as derivatives of aliphatic secondary and tertiary amines in which the aliphatic radical can be straight or branched chain and wherein one of the aliphatic substituents contains from about 8 to about 22 carbon atoms (preferably C₈-C₁₈) and one contains an anionic water solubilizing group, e.g., carboxy, sulfonate, sulfate, phosphate, or phosphonate. Examples are alkyl imino acetates, and iminodialkanoates and aminoalkanoates of the formulas RN[CH₂)ₘCO₂M]₂ and RNH(CH₂)ₘCO₂M wherein m is an intefer of from 1 to 4, R is a C₈-C₂₂ alkyl or alkenyl, and M is H, alkali metal, alkaline earth metal ammonium, or alkanolammonium. Also included are imidazolinium and ammonium derivatives. Specific examples of suitable amphoteric surfactants include sodium 3-dodecyl-aminopropionate, sodium 3-dodecylaminopropane sulfonate, N-alkyltaurines such as the one prepared by reacting dodecylamine with sodium isethionate according to the teaching of U.S. Pat. No. 2,658,072 which is incorporated herein by reference in its entirety; N-higher alkyl aspartic acids such as those produced according to the teaching of U.S. Pat. No. 2,438,091 which is incorporated herein by reference in its entirety; and the products sold under the trade name "Miranol" and described in U.S. Pat. No. 2,528,378, which is incorporated herein by reference in its entirety. Other examples of useful amphoterics include phosphates, such as coamidopropyl PG-dimonium chloride phosphate (commercially available as Monaquat PTC, from Mona Corp.).

Other amphoteric or zwitterionic surfactants useful herein include betaines. Examples of betaines include the higher alkyl betaines, such as coco dimethyl carboxymethyl betaine, lauryl dimethyl carboxymethyl betaine, lauryl dimethyl alphacarboxyethyl betaine, cetyl dimethyl carboxymethyl betaine, cetyl dimethyl betaine (available as Lonzaine 16SP from Lonza Corp.), lauryl bis-(2-hydroxyethyl)carboxymethyl betaine, stearyl bis-(2-hydroxypropyl)carboxy -methyl betaine, oleyl dimethyl gamma-carboxypropyl betaine, lauryl bis-(2-hydroxypropyl) -alpha-carboxyethyl betaine, coco dimethyl sulfopropyl betaine, stearyl dimethyl sulfopropyl betaine, lauryl dimethyl sulfoethyl betaine, lauryl bis-(2-hydroxyethyl)sulfopropyl betaine, and amidobetaines and amidosulfobetaines (wherein the RCONH(CH₂)₃ radical is attached to the nitrogen atom of the betaine), oleyl betaine (available as amphoteric Velvetex OLB-50 from Henkel), and cocamidopropyl betaine (available as Velvetex BK-35 and BA-35 from Henkel).

Other useful amphoteric and zwitterionic surfactants include the sultaines and hydroxysultaines such as cocamidopropyl hydroxysultaine (available as Mirataine CBS from Rhone-Poulenc), and the alkanoyl sarcosinates corresponding to the formula RCON(CH₃)CH₂CH₂CO₂M wherein R is alkyl or alkenyl of about 10 to about 20 carbon atoms, and M is a water-soluble cation such as ammonium, sodium, potassium and trialkanolamine (e.g., triethanolamine), a preferred example of which is sodium lauroyl sarcosinate.

### (3) Water

The preferred oil-in-water emulsion contains from about 10% to about 98%, preferably from about 20% to about 95%, more preferably from about 30% to about 90% water by weight of the liquid carrier.

The hydrophobic phase is dispersed in the continuous aqueous phase. The hydrophobic phase may contain water insoluble or partially soluble materials such as are known in the art, including but not limited to the silicones described herein in reference to silicone-in-water emulsions, and other oils and lipids such as described above in reference to emulsions.

The cosmetic compositions of the present invention, including but not limited to lotions and creams, may contain a dermatologically acceptable emollient. Such compositions preferably contain from about 1% to about 50% of the emollient. As used herein, "emollient" refers to a material useful for the prevention or relief of dryness, as well as for the protection of the skin. A wide variety of suitable emollients are known and may be used herein. Sagarin, Cosmetics, Science and Technology, 2nd Edition, Vol. 1, pp. 32-43 (1972), incorporated herein by reference, contains numerous examples of materials suitable as an emollient. A preferred emollient is glycerin. Glycerin is preferably used in an amount of from about 0.001 to about 30%, more preferably from about 0.01 to about 20%, still more preferably from about 0.1 to about 10%, e.g., 5%.

Lotions and creams according to the present invention generally contain a solution carrier system and one or more emollients. Lotions and creams typically contain from about 1% to about 50%, preferably from about 1% to about 20%, of emollient; from about 50% to about 90%, preferably from about 60% to about 80%, water; and the vitamin B3 compound and skin care active substance in the above described amounts. Creams are generally thicker than lotions.

Ointments of the present invention may contain animal or vegetable oils or semi-solid hydrocarbons (oleaginous); absorption ointment bases which absorb water to form emulsions; or water soluble carriers, e.g., a water soluble solution carrier. Ointments may further contain a thickening agent, such as described in Sagarin, Cosmetics, Science and Technology, 2nd Edition, Vol. 1, pp. 72-73 (1972), incorporated herein by reference, and/or an emollient. For example, an ointment may contain from about 2% to about 10% of an emollient; from about 0.1% to about 2% of a thickening agent; and the vitamin B3 compound and skin care active substance in the above described amounts.

Foundation carriers according to the present invention may contain a powder system selected from talc or mica, colorants, and the plant extract, with or without additional solvents. As used herein, the term "foundation" refers to a liquid, semi-liquid, semi-solid, or solid skin cosmetic which includes, but is not limited to lotions, creams, gels, pastes, cakes, and the like. Typically the foundation is used over a large area of the skin, such as the face, to provide a particular effect. Foundations are typically used to provide an adherent base for color cosmetics such as rouge, blusher, powder and the like, and tend to hide skin imperfections and impart a smooth, even appearance to the skin. Foundations in the present invention include a dermatologically acceptable carrier and may include conventional ingredients such as oils, colorants, pigments, emollients, fragrances, waxes, stabilizers, and the like. Exemplary carriers and such other ingredients which are suitable for use herein are described, for example, in PCT Application, WO 96/33689, to Canter, et al., published on Oct. 31, 1996 and U.K. Patent, GB 2274585, issued on Aug. 3, 1994.

The cosmetic composition of the invention may contain one or more additional skin care actives.

In a preferred embodiment, where the composition is to be in contact with human keratinous tissue, the additional components should be suitable for application to keratinous tissue, that is, when incorporated into the composition they are suitable for use in contact with human keratinous tissue without undue toxicity, incompatibility, instability, allergic response, and the like within the scope of sound medical judgment. The CTFA Cosmetic Ingredient Handbook, Second Edition (1992) describes a wide variety of nonlimiting cosmetic and pharmaceutical ingredients commonly used in the skin care industry, which are suitable for use in the compositions of the present invention. Examples of these ingredient classes include: abrasives, absorbents, aesthetic components such as fragrances, pigments, colorings/colorants, essential oils, skin sensates, astringents, etc. (e.g., clove oil, menthol, camphor, eucalyptus oil, eugenol, menthyl lactate, witch hazel distillate), anti-acne agents, anti-caking agents, antifoaming agents, antimicrobial agents (e.g., iodopropyl butylcarbamate), antioxidants, binders, biological additives, buffering agents, bulking agents, chelating agents, chemical additives, colorants, cosmetic astringents, cosmetic biocides, denaturants, drug astringents, external analgesics, film formers or materials, e.g., polymers, for aiding the film-forming properties and substantivity of the composition (e.g., copolymer of eicosene and vinyl pyrrolidone), opacifying agents, pH adjusters, propellants, reducing agents, sequestrants, skin bleaching and lightening agents (e.g., hydroquinone, kojic acid, ascorbic acid, magnesium ascorbyl phosphate, ascorbyl glucosamine, ascorbyl glucoside), skin-conditioning agents (e.g., humectants, including miscellaneous and occlusive), skin soothing and/or healing agents (e.g., panthenol and derivatives (e.g., ethyl panthenol), aloe vera, pantothenic acid and its derivatives, allantoin, bisabolol, and dipotassium glycyrrhizinate), skin treating agents, thickeners, and vitamins and derivatives thereof.

### Desquamation Actives

A safe and effective amount of a desquamation active may be added to the compositions of the present invention, more preferably from about 0.1% to about 10%, even more preferably from about 0.2% to about 5%, also preferably from about 0.5% to about 4%, by weight of the composition. Desquamation actives used in the present invention are favorable to the skin appearance. For example, the desquamation actives tend to improve the texture of the skin (e.g., smoothness). One desquamation system that is suitable for use herein contains sulfhydryl compounds and zwitterionic surfactants and is described in U.S. Pat. No. 5,681,852, to Bissett, incorporated herein by reference. Another desquamation system that is suitable for use herein contains salicylic acid and zwitterionic surfactants and is described in U.S. Pat. No. 5,652,228 to Bissett, incorporated herein by reference. Zwitterionic surfactants such as those described in these applications are also useful as desquamatory agents herein, with cetyl betaine being particularly preferred.

### Anti-Acne Actives

The compositions of the present invention may contain a safe and effective amount of one or more anti-acne actives. Examples of useful anti-acne actives include resorcinol, sulfur, salicylic acid, benzoyl peroxide, erythromycin, zinc, etc., which are described in further detail in U.S. Pat. No. 5,607,980, issued to McAtee et al, on Mar. 4, 1997.

### Anti-Wrinkle Actives/Anti-Atrophy Actives

The compositions of the present invention may further contain a safe and effective amount of one or more anti-wrinkle actives or anti-atrophy actives. Exemplary anti-wrinkle/anti-atrophy actives suitable for use in the compositions of the present invention include sulfur-containing D and L amino acids and their derivatives and salts, particularly the N-acetyl derivatives, a preferred example of which is N-acetyl-L-cysteine; thiols, e.g. ethane thiol; hydroxy acids (e.g., alpha-hydroxy acids such as lactic acid and glycolic acid or beta-hydroxy acids such as salicylic acid and salicylic acid derivatives such as the octanoyl derivative), phytic acid, lipoic acid; lysophosphatidic acid, skin peel agents (e.g., phenol and the like), vitamin B₃ compounds and retinoids which can improve the keratinous tissue appearance, especially in regulating keratinous tissue condition.

### a) Vitamin B₃ Compounds

The compositions of the present invention may contain a safe and effective amount of a vitamin B₃ compound. Vitamin B₃ compounds are useful for regulating skin condition as described in co-pending U.S. application Ser. No. 08/834,010, filed Apr. 11, 1997 (corresponding to international publication WO 97/39733 A1, published Oct. 30, 1997). When vitamin B₃ compounds are present in the compositions of the instant invention, the compositions preferably contain from about 0.01% to about 50%, more preferably from about 0.1% to about 10%, even more preferably from about 0.5% to about 10%, and still more preferably from about 1% to about 5%, still more preferably from about 2% to about 5%, by weight of the composition, of the vitamin B₃ compound.

Exemplary derivatives of the foregoing vitamin B₃ compounds include nicotinic acid esters, including non-vasodilating esters of nicotinic acid (e.g., tocopheryl nicotinate), nicotinyl amino acids, nicotinyl alcohol esters of carboxylic acids, nicotinic acid N-oxide and niacinamide N-oxide.

Examples of suitable vitamin B₃ compounds are well known in the art and are commercially available from a number of sources, e.g., the Sigma Chemical Company (St. Louis, Mo.); ICN Biomedicals, Inc. (Irvin, Calif.) and Aldrich Chemical Company (Milwaukee, Wis.).

The vitamin compounds may be included as the substantially pure material, or as an extract obtained by suitable physical and/or chemical isolation from natural (e.g., plant) sources.

### b) Retinoids

The compositions of the present invention may also contain a retinoid composition. As used herein, "retinoid" includes all natural and/or synthetic analogs of Vitamin A or retinol-like compounds which possess the biological activity of Vitamin A in the skin as well as the geometric isomers and stereoisomers of these compounds. The retinoid is preferably retinol, retinol esters (e.g., C₂-C₂₂ alkyl esters of retinol, including retinyl palmitate, retinyl acetate, retinyl propionate), retinal, and/or retinoic acid (including all-trans retinoic acid and/or 1,3-cis-retinoic acid), more preferably retinoids other than retinoic acid. These compounds are well known in the art and are commercially available from a number of sources, e.g., Sigma Chemical Company (St. Louis, Mo.), and Boerhinger Mannheim (Indianapolis, Ind.). Other retinoids which are useful herein are described in U.S. Pat. No. 4,677,120, issued Jun. 30, 1987 to Parish et al.; U.S. Pat. No. 4,885,311, issued Dec. 5, 1989 to Parish et al.; U.S. Pat. No. 5,049,584, issued Sep. 17, 1991 to Purcell et al.; U.S. Pat. No. 5,124,356, issued Jun. 23, 1992 to Purcell et al.; and U.S. Pat. No. Reissue 34,075, issued Sep. 22, 1992 to Purcell et al. Other suitable retinoids are tocopheryl-retinoate [tocopherol ester of retinoic acid (trans- or cis-), adapalene {6-[3-(1-adamantyl)-4-methoxyphenyl]-2-naphthoic acid}, and tazarotene (ethyl 6-[2-(4,4-dimethylthiochroman-6-yl)-ethynyl]nicotinate). Preferred retinoids are retinol, retinyl palmitate, retinyl acetate, retinyl propionate, retinal and combinations thereof.

The retinoid may be included as the substantially pure material, or as an extract obtained by suitable physical and/or chemical isolation from natural (e.g., plant) sources. The retinoid is preferably substantially pure, more preferably essentially pure.

The compositions of this invention may contain a safe and effective amount of the retinoid, such that the resultant composition is safe and effective for regulating keratinous tissue condition, preferably for regulating appearance and/or tactile feeling in skin, more preferably for regulating signs of skin aging. The compositions preferably contain from about 0.005% to about 2%, more preferably 0.01% to about 2%, retinoid. Retinol is preferably used in an amount of from or about 0.01% to or about 0.15%; retinol esters are preferably used in an amount of from about 0.01% to about 2% (e.g., about 1%); retinoic acids are preferably used in an amount of from about 0.01% to about 0.25%; tocopheryl-retinoate, adapalene, and tazarotene are preferably used in an amount of from about 0.01% to about 2%.

Where the compositions of the present invention contain both a retinoid and a Vitamin B₃ compound, the retinoid is preferably used in the above amounts, and the vitamin B₃ compound is preferably used in an amount of from about 0.1% to about 10%, more preferably from about 2% to about 5%.

### c) Hydroxy Acids

The compositions of the present invention may contain a safe and effective amount of a Hydroxy Acid. Preferred hydroxy acids for use in the compositions of the present invention include salicylic acid and salicylic acid derivatives. When present in the compositions of the present invention, salicylic acid is preferably used in an amount of from about 0.01% to about 50%, more preferably from about 0.1% to about 20%, even more preferably from about 0.1% to about 10%, still more preferably from about 0.5% to about 5%, and still more preferably from about 0.5% to about 2%.

### Peptides

Peptides, including but not limited to, di-, tri-, tetra-, and pentapeptides and derivatives thereof, may be included in the compositions of the present invention in amounts that are safe and effective. As used herein, "peptides" refers to both the naturally occurring peptides and synthesized peptides. Also useful herein are naturally occurring and commercially available compositions that contain peptides.

Suitable dipeptides for use herein include Carnosine (beta-ala-his). Suitable tripeptides for use herein include, gly-his-lys, arg-lys-arg, his-gly-gly. Preferred tripeptides and derivatives thereof include palmitoyl-gly-his-lys, which may be purchased as Biopeptide CL.RTM. (100 ppm of palmitoyl-gly-his-lys commerically available from Sederma, France); Peptide CK (arg-lys-arg); Peptide CK+ (ac-arg-lys-arg-NH₂); and a copper derivative of his-gly-gly sold commercially as Iamin, from Sigma (St.Louis, Mo.). Suitable tetrapeptides for use herein include Peptide E, arg-ser-arg-lys (SEQ ID NO:1). Suitable pentapeptides for use herein include lys-thr-thr-lys-ser. A preferred commercially available pentapeptide derivative composition is Matrixyl.RTM., which contains 100 ppm palmitoyl-lys-thr-thr-lys-ser (SEQ ID NO:2, commercially available from Sederma France).

Preferably, the peptide is selected from palmitoyl-lys-thr-thr-lys-ser, palmitoyl-gly-his-lys, beta-ala-his, their derivatives, and combinations thereof. More preferably, the peptide is selected from palmitoyl-lys-thr-thr-lys-ser, palmitoyl-gly-his-lys, their derivatives, and combinations thereof. Even more preferably, the peptide is selected from palmitoyl-lys-thr-thr-lys-ser and derivatives thereof.

When included in the present compositions, peptides are preferably included in amounts of from about 1x10⁻⁶ % to about 10%, more preferably from about 1x10⁻⁶ % to about 0.1%, even more preferably from about 1x10⁻⁵ % to about 0.01%, by weight of the composition. In certain compositions where the peptide is Carnosine.RTM., the compositions preferably contain from about 0.1% to about 5%, by weight of the composition, of such peptides. In other embodiments wherein the peptide-containing compositions, Matrixyl.RTM., and/or Biopeptide CL.RTM. are included, the compositions preferably contain from about 0.1% to about 10%, by weight compositions, of Matrixyl.RTM. and/or Biopeptide CL.RTM. peptide-containing compositions.

### Anti-Oxidants/Radical Scavengers

The compositions of the present invention may include a safe and effective amount of an anti-oxidant/radical scavenger. The anti-oxidant/radical scavenger is especially useful for providing protection against UV radiation which can cause increased scaling or texture changes in the stratum corneum and against other environmental agents which can cause skin damage.

A safe and effective amount of an anti-oxidant/radical scavenger may be added to the compositions of the subject invention, preferably from about 0.1% to about 10%, more preferably from about 1% to about 5%, of the composition.

Anti-oxidants/radical scavengers such as ascorbic acid (vitamin C) and its salts, ascorbyl esters of fatty acids, ascorbic acid derivatives (e.g., magnesium ascorbyl phosphate, sodium ascorbyl phosphate, ascorbyl sorbate), tocopherol (vitamin E), tocopherol sorbate, tocopherol acetate, other esters of tocopherol, butylated hydroxy benzoic acids and their salts, 6-hydroxy-2,5,7,8-tetramethylchroman-2-carboxylic acid (commercially available under the tradename Trolox.sup.R), gallic acid and its alkyl esters, especially propyl gallate, uric acid and its salts and alkyl esters, sorbic acid and its salts, lipoic acid, amines (e.g., N,N-diethylhydroxylamine, amino-guanidine), sulfhydryl compounds (e.g., glutathione), dihydroxy fumaric acid and its salts, lycine pidolate, arginine pilolate, nordihydroguaiaretic acid, bioflavonoids, curcumin, lysine, methionine, proline, superoxide dismutase, silymarin, tea extracts, grape skin/seed extracts, melanin, and rosemary extracts may be used. Preferred anti-oxidants/radical scavengers are selected from tocopherol sorbate and other esters of tocopherol, more preferably tocopherol sorbate. For example, the use of tocopherol sorbate in topical compositions and applicable to the present invention is described in U.S. Pat. No. 4,847,071, issued on Jul. 11, 1989 to Donald L. Bissett, Rodney D. Bush and Ranjit Chatterjee.

### Chelators

The compositions of the present invention may also contain a safe and effective amount of a chelator or chelating agent. As used herein, "chelator" or "chelating agent" means an active agent capable of removing a metal ion from a system by forming a complex so that the metal ion cannot readily participate in or catalyze chemical reactions. The inclusion of a chelating agent is especially useful for providing protection against UV radiation which can contribute to excessive scaling or skin texture changes and against other environmental agents which can cause skin damage.

A safe and effective amount of a chelating agent may be added to the compositions of the subject invention, preferably from about 0. 1% to about 10%, more preferably from about 1% to about 5%, of the composition. Exemplary chelators that are useful herein are disclosed in U.S. Pat. No. 5,487,884, issued Jan. 30, 1996 to Bissett et al.; International Publication No. 91/16035, Bush et al., published Oct. 31, 1995; and International Publication No. 91/16034, Bush et al., published Oct. 31, 1995. Preferred chelators useful in compositions of the subject invention are furildioxime, furilmonoxime, and derivatives thereof.

### Flavonoids

The compositions of the present invention may optionally contain a flavonoid compound. Flavonoids are broadly disclosed in U.S. Pat. Nos. 5,686,082 and 5,686,367, both of which are herein incorporated by reference. Flavonoids suitable for use in the present invention are flavanones selected from unsubstituted flavanones, mono-substituted flavanones, and mixtures thereof; chalcones selected from unsubstituted chalcones, mono-substituted chalcones, di-substituted chalcones, tri-substituted chalcones, and mixtures thereof; flavones selected from unsubstituted flavones, mono-substituted flavones, di-substituted flavones, and mixtures thereof; one or more isoflavones; coumarins selected from unsubstituted coumarins, mono-substituted coumarins, di-substituted coumarins, and mixtures thereof; chromones selected from unsubstituted chromones, mono-substituted chromones, di-substituted chromones, and mixtures thereof; one or more dicoumarols; one or more chromanones; one or more chromanols; isomers (e.g., cis/trans isomers) thereof; and mixtures thereof. By the term "substituted" as used herein means flavonoids wherein one or more hydrogen atom of the flavonoid has been independently replaced with hydroxyl, C₁-C₈ alkyl, C₁-C₄ alkoxyl, 0-glycoside, and the like or a mixture of these substituents.

Examples of suitable flavonoids include, but are not limited to, unsubstituted flavanone, mono-hydroxy flavanones (e.g., 2'-hydroxy flavanone, 6-hydroxy flavanone, 7-hydroxy flavanone, etc.), mono-alkoxy flavanones (e.g., 5-methoxy flavanone, 6-methoxy flavanone, 7-methoxy flavanone, 4'-methoxy flavanone, etc.), unsubstituted chalcone (especially unsubstituted trans-chalcone), mono-hydroxy chalcones (e.g., 2'-hydroxy chalcone, 4'-hydroxy chalcone, etc.), di-hydroxy chalcones (e.g., 2',4-dihydroxy chalcone, 2',4'-dihydroxy chalcone, 2,2'-dihydroxy chalcone, 2',3-dihydroxy chalcone, 2',5'-dihydroxy chalcone, etc.), and tri-hydroxy chalcones (e.g., 2',3',4'-trihydroxy chalcone, 4,2',4'-trihydroxy chalcone, 2,2',4'-trihydroxy chalcone, etc.), unsubstituted flavone, 7,2'-dihydroxy flavone, 3',4'-dihydroxy naphthoflavone, 4'-hydroxy flavone, 5,6-benzoflavone, and 7,8-benzoflavone, unsubstituted isoflavone, daidzein (7,4'-dihydroxy isoflavone), 5,7-dihydroxy-4'-methoxy isoflavone, soy isoflavones (a mixture extracted from soy), unsubstituted coumarin, 4-hydroxy coumarin, 7-hydroxy coumarin, 6-hydroxy-4-methyl coumarin, unsubstituted chromone, 3-formyl chromone, 3-formyl-6-isopropyl chromone, unsubstituted dicoumarol, unsubstituted chromanone, unsubstituted chromanol, and mixtures thereof.

Preferred for use herein are unsubstituted flavanone, methoxy flavanones, unsubstituted chalcone, 2',4-dihydroxy chalcone, and mixtures thereof. More preferred are unsubstituted flavanone, unsubstituted chalcone (especially the trans isomer), and mixtures thereof.

They can be synthetic materials or obtained as extracts from natural sources (e.g., plants). The naturally sourced material can also further be derivatized (e.g., an ester or ether derivative prepared following extraction from a natural source). Flavonoid compounds useful herein are commercially available from a number of sources, e.g., Indofine Chemical Company, Inc. (Somerville, N.J.), Steraloids, Inc. (Wilton, N.H.), and Aldrich Chemical Company, Inc. (Milwaukee, Wis.).

Mixtures of the above flavonoid compounds may also be used.

The herein described flavonoid compounds are preferably present in the instant invention at concentrations of from about 0.01% to about 20%, more preferably from about 0.1% to about 10%, and still more preferably from about 0.5% to about 5%.

### Anti-Inflammatory Agents

A safe and effective amount of an anti-inflammatory agent may be added to the compositions of the present invention, preferably from about 0.1% to about 10%, more preferably from about 0.5% to about 5%, of the composition. The anti-inflammatory agent enhances the skin appearance benefits of the present invention, e.g., such agents contribute to a more uniform and acceptable skin tone or color. The exact amount of anti-inflammatory agent to be used in the composition will depend on the particular anti-inflammatory agent utilized since such agents vary widely in potency.

Steroidal anti-inflammatory agents, including but not limited to, corticosteroids such as hydrocortisone, hydroxyltriamcinolone, alpha-methyl dexamethasone, dexamethasone- phosphate, beclomethasone dipropionates, clobetasol valerate, desonide, desoxymethasone, desoxycorticosterone acetate, dexamethasone, dichlorisone, diflorasone diacetate, diflucortolone valerate, fluadrenolone, fluclorolone acetonide, fludrocortisone, flumethasone pivalate, fluosinolone acetonide, fluocinonide, flucortine butylesters, fluocortolone, fluprednidene (fluprednylidene) acetate, flurandrenolone, halcinonide, hydrocortisone acetate, hydrocortisone butyrate, methylprednisolone, triamcinolone acetonide, cortisone, cortodoxone, flucetonide, fludrocortisone, difluorosone diacetate, fluradrenolone, fludrocortisone, diflurosone diacetate, fluradrenolone acetonide, medrysone, amcinafel, amcinafide, betamethasone and the balance of its esters, chloroprednisone, chlorprednisone acetate, clocortelone, clescinolone, dichlorisone, diflurprednate, flucloronide, flunisolide, fluoromethalone, fluperolone, fluprednisolone, hydrocortisone valerate, hydrocortisone cyclopentylpropionate, hydrocortamate, meprednisone, paramethasone, prednisolone, prednisone, beclomethasone dipropionate, triamcinolone, and mixtures thereof may be used. The preferred steroidal anti-inflammatory for use is hydrocortisone.

A second class of anti-inflammatory agents which is useful in the compositions of the present invention includes the nonsteroidal anti-inflammatory agents. The variety of compounds encompassed by this group are well-known to those skilled in the art. For detailed disclosure of the chemical structure, synthesis, side effects, etc. of non-steroidal anti-inflammatory agents, one may refer to standard texts, including Anti-inflammatory and Anti-Rheumatic Drugs, K. D. Rainsford, Vol. I-III, CRC Press, Boca Raton, (1985), and Anti-inflammatory Agents, Chemistry and Pharmacology, 1, R. A. Scherrer, et al., Academic Press, New York (1974).

Specific non-steroidal anti-inflammatory agents useful in the composition of the invention include, but are not limited to: 1) the oxicams, such as piroxicam, isoxicam, tenoxicam, sudoxicam, and CP-14,304; 2) the salicylates, such as aspirin, disalcid, benorylate, trilisate, safapryn, solprin, diflunisal, and fendosal; 3) the acetic acid derivatives, such as diclofenac, fenclofenac, indomethacin, sulindac, tolmetin, isoxepac, furofenac, tiopinac, zidometacin, acematacin, fentiazac, zomepirac, clindanac, oxepinac, felbinac, and ketorolac; 4) the fenamates, such as mefenamic, meclofenamic, flufenamic, niflumic, and tolfenamic acids; 5) the propionic acid derivatives, such as ibuprofen, naproxen, benoxaprofen, flurbiprofen, ketoprofen, fenoprofen, fenbufen, indopropfen, pirprofen, carprofen, oxaprozin, pranoprofen, miroprofen, tioxaprofen, suprofen, alminoprofen, and tiaprofenic; and 6) the pyrazoles, such as phenylbutazone, oxyphenbutazone, feprazone, azapropazone, and trimethazone.

Mixtures of these non-steroidal anti-inflammatory agents may also be employed, as well as the dermatologically acceptable salts and esters of these agents. For example, etofenamate, a flufenamic acid derivative, is particularly useful for topical application. Of the nonsteroidal anti-inflammatory agents, ibuprofen, naproxen, flufenamic acid, etofenamate, aspirin, mefenamic acid, meclofenamic acid, piroxicam and felbinac are preferred; ibuprofen, naproxen, ketoprofen, etofenamate, aspirin and flufenamic acid are more preferred.

Finally, so-called "natural" anti-inflammatory agents are useful in the composition of the present invention. Such agents may suitably be obtained as an extract by suitable physical and/or chemical isolation from natural sources (e.g., plants, fungi, by-products of microorganisms) or can be synthetically prepared. For example, candelilla wax, bisabolol (e.g., alpha bisabolol), aloe vera, plant sterols (e.g., phytosterol), Manjistha (extracted from plants in the genus Rubia, particularly Rubia Cordifolia), and Guggal (extracted from plants in the genus Commiphora, particularly Commiphora Mukul), kola extract, chamomile, red clover extract, and sea whip extract, may be used.

Additional anti-inflammatory agents useful herein include compounds of the Licorice (the plant genus/species Glycyrrhiza glabra) family, including glycyrrhetic acid, glycyrrhizic acid, and derivatives thereof (e.g., salts and esters). Suitable salts of the foregoing compounds include metal and ammonium salts. Suitable esters include C₂-C₂₄ saturated or unsaturated esters of the acids, preferably C₁₀-C₂₄, more preferably C₁₆-C₂₄. Specific examples of the foregoing include oil soluble licorice extract, the glycyrrhizic and glycyrrhetic acids themselves, monoammonium glycyrrhizinate, monopotassium glycyrrhizinate, dipotassium glycyrrhizinate, 1-beta-glycyrrhetic acid, stearyl glycyrrhetinate, and 3-stearyloxy-glycyrrhetinic acid, and disodium 3-succinyloxy-beta-glycyrrhetinate. Stearyl glycyrrhetinate is preferred.

### Anti-Cellulite Agents

The compositions of the present invention may also contain a safe and effective amount of an anti-cellulite agent. Suitable agents may include, but are not limited to, xanthine compounds (e.g., caffeine, theophylline, theobromine, and aminophylline).

### Topical Anesthetics

The compositions of the present invention may also contain a safe and effective amount of a topical anesthetic. Examples of topical anesthetic drugs include benzocaine, lidocaine, bupivacaine, chlorprocaine, dibucaine, etidocaine, mepivacaine, tetracaine, dyclonine, hexylcaine, procaine, cocaine, ketamine, pramoxine, phenol, and pharmaceutically acceptable salts thereof.

### Tanning Actives

The compositions of the present invention may contain a tanning active. When present, it is preferable that the compositions contain from about 0.1 % to about 20%, more preferably from about 2% to about 7%, and still more preferably from about 3% to about 6%, by weight of the composition, of dihydroxyacetone as an artificial tanning active.

Dihydroxyacetone, which is also known as DHA or 1,3-dihydroxy-2-propanone, is a white to off-white, crystalline powder. This material can be represented by the chemical formula C₃H₆O₃. The compound can exist as a mixture of monomers and dimers, with the dimers predominating in the solid crystalline state. Upon heating or melting, the dimers break down to yield the monomers. This conversion of the dimeric form to the monomeric form also occurs in aqueous solution. Dihydroxyacetone is also known to be more stable at acidic pH values. See The Merck Index, Tenth Edition, entry 3167, p. 463 (1983), and "Dihydroxyacetone for Cosmetics", E. Merck Technical Bulletin, 03-304 110, 319 897, 180 588.

### Skin Lightening Agents

The compositions of the present invention may contain a skin lightening agent. When used, the compositions preferably contain from about 0.1% to about 10%, more preferably from about 0.2% to about 5%, also preferably from about 0.5% to about 2%, by weight of the composition, of a skin lightening agent. Suitable skin lightening agents include those known in the art, including kojic acid, arbutin, ascorbic acid and derivatives thereof (e.g., magnesium ascorbyl phosphate, sodium ascorbyl phosphate or ascorbyl glucoside), and extracts (e.g., mulberry extract, placental extract). Skin lightening agents suitable for use herein also include those described in the PCT publication No. 95/34280, in the name of Hillebrand, corresponding to PCT Application No. U.S. 95/07432, filed Jun. 12, 1995; and co-pending U.S. application Ser. No. 08/390, 152 filed in the names of Kvalnes, Mitchell A. DeLong, Barton J. Bradbury, Curtis B. Motley, and John D. Carter, corresponding to PCT Publication No. 95/23780, published Sep. 8, 1995.

### Skin Soothing and Skin Healing Actives

The compositions of the present invention may comprise a skin soothing or skin healing active. Skin soothing or skin healing actives suitable for use herein include panthenoic acid derivatives (including panthenol, dexpanthenol, ethyl panthenol), aloe vera, allantoin, bisabolol, and dipotassium glycyrrhizinate. A safe and effective amount of a skin soothing or skin healing active may be added to the present composition, preferably, from about 0.1% to about 30%, more preferably from about 0.5% to about 20%, still more preferably from about 0.5% to about 10%, by weight of the composition formed.

### Antimicrobial and Antifungal Actives

The compositions of the present invention may contain an antimicrobial or antifungal active. Such actives are capable of destroying microbes, preventing the development of microbes or preventing the pathogenic action of microbes. A safe and effective amount of an antimicrobial or antifungal active may be added to the present compositions, preferably, from about 0.001% to about 10%, more preferably from about 0.01% to about 5%, and still more preferably from about 0.05% to about 2%.

Examples of antimicrobial and antifungal actives include B-lactam drugs, quinolone drugs, ciprofloxacin, norfloxacin, tetracycline, erythromycin, amikacin, 2,4,4'-trichloro-2'-hydroxy diphenyl ether, 3,4,4'-trichlorobanilide, phenoxyethanol, phenoxy propanol, phenoxyisopropanol, doxycycline, capreomycin, chlorhexidine, chlortetracycline, oxytetracycline, clindamycin, ethambutol, hexamidine isethionate, metronidazole, pentamidine, gentamicin, kanamycin, lineomycin, methacycline, methenamine, minocycline, neomycin, netilmicin, paromomycin, streptomycin, tobramycin, miconazole, tetracycline hydrochloride, erythromycin, zinc erythromycin, erythromycin estolate, erythromycin stearate, amikacin sulfate, doxycycline hydrochloride, capreomycin sulfate, chlorhexidine gluconate, chlorhexidine hydrochloride, chlortetracycline hydrochloride, oxytetracycline hydrochloride, clindamycin hydrochloride, ethambutol hydrochloride, metronidazole hydrochloride, pentamidine hydrochloride, gentamicin sulfate, kanamycin sulfate, lineomycin hydrochloride, methacycline hydrochloride, methenamine hippurate, methenamine mandelate, minocycline hydrochloride, neomycin sulfate, netilmicin sulfate, paromomycin sulfate, streptomycin sulfate, tobramycin sulfate, miconazole hydrochloride, ketaconazole, amanfadine hydrochloride, amanfadine sulfate, octopirox, parachlorometa xylenol, nystatin, tolnaftate, zinc pyrithione and clotrimazole.

Preferred examples of actives useful herein include those selected from salicylic acid, benzoyl peroxide, 3-hydroxy benzoic acid, glycolic acid, lactic acid, 4-hydroxy benzoic acid, acetyl salicylic acid, 2-hydroxybutanoic acid, 2-hydroxypentanoic acid, 2-hydroxyhexanoic acid, cis-retinoic acid, trans-retinoic acid, retinol, phytic acid, N-acetyl-L-cysteine, lipoic acid, azelaic acid, arachidonic acid, benzoylperoxide, tetracycline, ibuprofen, naproxen, hydrocortisone, acetominophen, resorcinol, phenoxyethanol, phenoxypropanol, phenoxyisopropanol, 2,4,4'-trichloro-2'-hydroxy diphenyl ether, 3,4,4'-trichlorocarbanilide, octopirox, lidocaine hydrochloride, clotrimazole, miconazole, ketoconazole, neocycin sulfate, and mixtures thereof.

### Sunscreen Actives

Exposure to ultraviolet light can result in excessive scaling and texture changes of the stratum corneum. Therefore, the composition of the present invention may optionally contain a sunscreen active. As used herein, "sunscreen active" includes both sunscreen agents and physical sunblocks. Suitable sunscreen actives may be organic or inorganic.

Inorganic sunscreen actives useful herein include the following metallic oxides; titanium dioxide having an average primary particle size of from about 15 nm to about 100 nm, zinc oxide having an average primary particle size of from about 15 nm to about 150 nm, zirconium oxide having an average primary particle size of from about 15 nm to about 150 nm, iron oxide having an average primary particle size of from about 15 nm to about 500 nm, and mixtures thereof. When used herein, the inorganic sunscreen actives are present in the amount of from about 0.1% to about 20%, preferably from about 0.5% to about 10%, more preferably from about 1% to about 5%, by weight of the composition.

A wide variety of conventional organic sunscreen actives are suitable for use herein. Sagarin, et al., at Chapter VIII, pages 189 et seq., of Cosmetics Science and Technology (1972), discloses numerous suitable actives. Specific suitable sunscreen actives include, for example: p-aminobenzoic acid, its salts and its derivatives (ethyl, isobutyl, glyceryl esters; p-dimethylaminobenzoic acid); anthranilates (i.e., o-amino-benzoates; methyl, menthyl, phenyl, benzyl, phenylethyl, linalyl, terpinyl, and cyclohexenyl esters); salicylates (amyl, phenyl, octyl, benzyl, menthyl, glyceryl, and di-pro-pyleneglycol esters); cinnamic acid derivatives (menthyl and benzyl esters, a-phenyl cinnamonitrile; butyl cinnamoyl pyruvate); dihydroxycinnamic acid derivatives (umbelliferone, methylumbelliferone, methylaceto-umbelliferone); trihydroxy- cinnamic acid derivatives (esculetin, methylesculetin, daphnetin, and the glucosides, esculin and daphnin); hydrocarbons (diphenylbutadiene, stilbene); dibenzalacetone and benzalacetophenone; naphtholsulfonates (sodium salts of 2-naphthol-3,6-disulfonic and of 2-naphthol-6,8-disulfonic acids); di-hydroxynaphthoic acid and its salts; o- and p-hydroxybiphenyldisulfonates; coumarin derivatives (7-hydroxy, 7-methyl, 3-phenyl); diazoles (2-acetyl-3-bromoindazole, phenyl benzoxazole, methyl naphthoxazole, various aryl benzothiazoles); quinine salts (bisulfate, sulfate, chloride, oleate, and tannate); quinoline derivatives (8-hydroxyquinoline salts, 2-phenylquinoline); hydroxy- or methoxy-substituted benzophenones; uric and violuric acids; tannic acid and its derivatives (e.g., hexaethylether); (butyl carbotol) (6-propyl piperonyl) ether; hydroquinone; benzophenones (oxybenzene, sulisobenzone, dioxybenzone, benzoresorcinol, 2,2',4,4'-tetrahydroxybenzophenone, 2,2'-dihydroxy-4,4'-dimethoxybenzophenone, octabenzone; 4-isopropyldibenzoylmethane; butylmethoxydibenzoylmethane; etocrylene; octocrylene; [3-(4'-methylbenzylidene bornan- 2-one), terephthalylidene dicamphor sulfonic acid and 4-isopropyl-di-benzoylmethane.

Of these, 2-ethylhexyl-p-methoxycinnamate (commercially available as PARSOL MCX), 4,4'-t-butyl methoxydibenzoyl-methane (commercially available as PARSOL 1789), 2-hydroxy-4-methoxybenzophenone, octyldimethyl-p-aminobenzoic acid, digalloyltrioleate, 2,2-dihydroxy-4-methoxybenzophenone, ethyl-4-(bis(hydroxy-propyl))aminobenzoate, 2-ethylhexyl-2-cyano-3,3-diphenylacrylate, 2-ethylhexyl-salicylate, glyceryl-p-aminobenzoate, 3,3,5-tri-methylcyclohexylsalicylate, methylanthranilate, p-dimethyl-aminobenzoic acid or aminobenzoate, 2-ethylhexyl-p-dimethyl-amino-benzoate, 2-phenylbenzimidazole-5-sulfonic acid, 2-(p-dimethylaminophenyl)-5-sulfonicbenzoxazoic acid, octocrylene and mixtures of these compounds, are preferred.

More preferred organic sunscreen actives useful in the compositions are 2-ethylhexyl-p-methoxycinnamate, butylmethoxydibenzoyl-methane, 2-hydroxy-4-methoxybenzo-phenone, 2-phenylbenzimidazole-5-sulfonic acid, octyldimethyl-p-aminobenzoic acid, octocrylene and mixtures thereof.

Also particularly useful in the compositions of the invention are sunscreen actives such as those disclosed in U.S. Pat. No. 4,937,370 issued to Sabatelli on Jun. 26, 1990, and U.S. Pat. No. 4,999,186 issued to Sabatelli & Spirnak on Mar. 12, 1991. The sunscreening agents disclosed therein have, in a single molecule, two distinct chromophore moieties which exhibit different ultra-violet radiation absorption spectra. One of the chromophore moieties absorbs predominantly in the UVB radiation range and the other absorbs strongly in the UVA radiation range.

Preferred members of this class of sunscreening agents are 4-N,N-(2-ethyhexyl)methyl-aminobenzoic acid ester of 2,4-dihydroxybenzophenone; N,N-di-(2-ethylhexyl)-4-aminobenzoic acid ester with 4-hydroxydibenzoylmethane; 4-N,N-(2-ethylhexyl)methyl-aminobenzoic acid ester with 4-hydroxydibenzoylmethane; 4-N,N-(2-ethylhexyl)methyl-aminobenzoic acid ester of 2-hydroxy-4-(2-hydroxyethoxy)benzophenone;
4-N,N-(2-ethylhexyl)-methylaminobenzoic acid ester of 4-(2-hydroxyethoxy)dibenzoylmethane; N,N-di-(2-ethylhexyl)-4-aminobenzoic acid ester of 2-hydroxy-4-(2-hydroxyethoxy)benzophenone; and
N,N-di-(2-ethylhexyl)-4-aminobenzoic acid ester of 4-(2-hydroxyethoxy)dibenzoylmethane and mixtures thereof.

Especially preferred sunscreen actives include 4,4'-t-butylmethoxydibenzoylmethane, 2-ethylhexyl-p-methoxycinnamate, phenyl benzimidazole sulfonic acid, and octocrylene.

A safe and effective amount of the organic sunscreen active is used, typically from about 1% to about 20%, more typically from about 2% to about 10% by weight of the composition. Exact amounts will vary depending upon the sunscreen or sunscreens chosen and the desired Sun Protection Factor (SPF).

### Particulate Material

The compositions of the present invention may contain a particulate material, preferably a metallic oxide. These particulates can be coated or uncoated, charged or uncharged. Charged particulate materials are disclosed in U.S. Pat. No. 5,997,887, to Ha, et al., incorporated herein by reference. Particulate materials useful herein include; bismuth oxychloride, iron oxide, mica, mica treated with barium sulfate and TiO2, silica, nylon, polyethylene, talc, styrene, polypropylene, ethylene/acrylic acid copolymer, sericite, titanium dioxide, bismuth oxychloride, iron oxide, aluminum oxide, silicone resin, barium sulfate, calcium carbonate, cellulose acetate, polymethyl methacrylate, and mixtures thereof.

Inorganic particulate materials, e.g., TiO2, ZnO, or ZrO2 are commercially available from a number of sources. One example of a suitable particulate material contains the material available from U.S. Cosmetics (TRONOX TiO2 series, SAT-T CR837, a rutile TiO2). Preferably, particulate materials are present in the composition in levels of from about 0.01% to about 2%, more preferably from about 0.05% to about 1.5%, still more preferably from about 0.1% to about 1%, by weight of the composition.

### Conditioning Agents

The composition of the present invention may contain a conditioning agent selected from humectants, moisturizers, or skin conditioners. A variety of these materials can be employed and each can be present at a level of from about 0.01% to about 20%, more preferably from about 0.1% to about 10%, and still more preferably from about 0.5% to about 7% by weight of the composition. These materials include, but are not limited to, guanidine; urea; glycolic acid and glycolate salts (e.g. ammonium and quaternary alkyl ammonium); salicylic acid; lactic acid and lactate salts (e.g., ammonium and quaternary alkyl ammonium); aloe vera in any of its variety of forms (e.g., aloe vera gel); polyhydroxy alcohols such as sorbitol, mannitol, xylitol, erythritol, glycerol, hexanetriol, butanetriol, propylene glycol, butylene glycol, hexylene glycol and the like; polyethylene glycols; sugars (e.g., melibiose) and starches; sugar and starch derivatives (e.g., alkoxylated glucose, fucose, glucosamine); hyaluronic acid; lactamide monoethanolamine; acetamide monoethanolamine; panthenol; allantoin; and mixtures thereof. Also useful herein are the propoxylated glycerols described in U.S. Pat. No. 4,976,953, to Orr et al, issued Dec. 11,1990.

Also useful are various C₁-C₃₀ monoesters and polyesters of sugars and related materials. These esters are derived from a sugar or polyol moiety and one or more carboxylic acid moieties. Such ester materials are further described in, U.S. Pat. No. 2,831,854, U.S. Pat. No. 4,005,196, to Jandacek, issued Jan. 25, 1977; U.S. Pat. No. 4,005,195, to Jandacek, issued Jan. 25, 1977, U.S. Pat. No. 5,306,516, to Letton et al, issued Apr. 26, 1994; U.S. Pat. No. 5,306,515, to Letton et al, issued Apr. 26, 1994; U.S. Pat. No. 5,305,514, to Letton et al, issued Apr. 26, 1994; U.S. Pat. No. 4,797,300, to Jandacek et al, issued Jan. 10, 1989; U.S. Pat. No. 3,963,699, to Rizzi et al, issued Jun. 15, 1976; U.S. Pat. No. 4,518,772, to Volpenhein, issued May 21, 1985; and U.S. Pat. No. 4,517,360, to Volpenhein, issued May 21, 1985.

Preferably, the conditioning agent is selected from urea, guanidine, sucrose polyester, panthenol, dexpanthenol, allantoin, and combinations thereof.

### Structuring Agents

The compositions hereof, and especially the emulsions hereof, may contain a structuring agent. Structuring agents are particularly preferred in the oil-in-water emulsions of the present invention. Without being limited by theory, it is believed that the structuring agent assists in providing rheological characteristics to the composition which contribute to the stability of the composition. For example, the structuring agent tends to assist in the formation of the liquid crystalline gel network structures. The structuring agent may also function as an emulsifier or surfactant. Preferred compositions of this invention contain from about 0.1% to about 20%, more preferably from about 0.1% to about 10%, still more preferably from about 0.5% to about 9%, of one or more structuring agents.

Preferred structuring agents are those having an HLB of from about 1 to about 8 and having a melting point of at least about 45°C. Suitable structuring agents are those selected from saturated C₁₄ to C₃₀ fatty alcohols, saturated C₁₆-C₃₀ fatty alcohols containing from about 1 to about 5 moles of ethylene oxide, saturated C₁₆-C₃₀ diols, saturated C₁₆-C₃₀ monoglycerol ethers, saturated C₁₆-C₃₀ hydroxy fatty acids, C₁₄-C₃₀ hydroxylated and nonhydroxylated saturated fatty acids, C₁₄-C₃₀ saturated ethoxylated fatty acids, amines and alcohols containing from about 1 to about 5 moles of ethylene oxide diols, C₁₄-C₃₀ saturated glyceryl mono esters with a monoglyceride content of at least 40%, C₁₄-C₃₀ saturated polyglycerol esters having from about 1 to about 3 alkyl group and from about 2 to about 3 saturated glycerol units, C₁₄-C₃₀ glyceryl mono ethers, C₁₄-C₃₀ sorbitan mono/diesters, C₁₄-C₃₀ saturated ethoxylated sorbitan mono/diesters with about 1 to about 5 moles of ethylene oxide, C₁₄-C₃₀ saturated methyl glucoside esters, C₁₄-C₃₀ saturated sucrose mono/diesters, C₁₄-C₃₀ saturated ethoxylated methyl glucoside esters with about 1 to about 5 moles of ethylene oxide, C₁₄-C₃₀ saturated polyglucosides having an average of between 1 to 2 glucose units and mixtures thereof, having a melting point of at least about 45°C.

The preferred structuring agents of the present invention are selected from stearic acid, palmitic acid, stearyl alcohol, cetyl alcohol, behenyl alcohol, stearic acid, palmitic acid, the polyethylene glycol ether of stearyl alcohol having an average of about 1 to about 5 ethylene oxide units, the polyethylene glycol ether of cetyl alcohol having an average of about 1 to about 5 ethylene oxide units, and mixtures thereof. More preferred structuring agents of the present invention are selected from stearyl alcohol, cetyl alcohol, behenyl alcohol, the polyethylene glycol ether of stearyl alcohol having an average of about 2 ethylene oxide units (steareth-2), the polyethylene glycol ether of cetyl alcohol having an average of about 2 ethylene oxide units, and mixtures thereof. Even more preferred structuring agents are selected from stearic acid, palmitic acid, stearyl alcohol, cetyl alcohol, behenyl alcohol, steareth-2, and mixtures thereof.

### Thickening Agent (Including Thickeners and Gelling Agents)

The compositions of the present invention can contain one or more thickening agents, preferably from about 0.1% to about 5%, more preferably from about 0.1% to about 4%, and still more preferably from about 0.25% to about 3%, by weight of the composition.

Nonlimiting classes of thickening agents include those selected from the following:

### a) Carboxylic Acid Polymers

These polymers are crosslinked compounds containing one or more monomers derived from acrylic acid, substituted acrylic acids, and salts and esters of these acrylic acids and the substituted acrylic acids, wherein the crosslinking agent contains two or more carbon-carbon double bonds and is derived from a polyhydric alcohol. Polymers useful in the present invention are more fully described in U.S. Pat. No. 5,087,445, to Haffey et al, issued Feb. 11, 1992; U.S. Pat. No. 4,509,949, to Huang et al, issued Apr. 5, 1985; U.S. Pat. No. 2,798,053, to Brown, issued Jul. 2, 1957; and in CTFA International Cosmetic Ingredient Dictionary, Fourth Edition, 1991, pp. 12 and 80.

Examples of commercially available carboxylic acid polymers useful herein include the carbomers, which are homopolymers of acrylic acid crosslinked with allyl ethers of sucrose or pentaerytritol. The carbomers are available as the Carbopol.RTM. 900 series from B.F. Goodrich (e.g., Carbopol.RTM. 954). In addition, other suitable carboxylic acid polymeric agents include copolymers of C₁₀₋₃₀ alkyl acrylates with one or more monomers of acrylic acid, methacrylic acid, or one of their short chain (i.e., C₁₋₄ alcohol) esters, wherein the crosslinking agent is an allyl ether of sucrose or pentaerytritol. These copolymers are known as acrylates/C₁₀₋₃₀ alkyl acrylate crosspolymers and are commercially available as Carbopol.RTM. 1342, Carbopol.RTM. 1382, Pemulen TR-1, and Pemulen TR-2, from B.F. Goodrich. In other words, examples of carboxylic acid polymer thickeners useful herein are those selected from carbomers, acrylates/C₁₀-C₃₀ alkyl acrylate crosspolymers, and mixtures thereof.

### b) Crosslinked Polyacrylate Polymers

The compositions of the present invention can optionally contain crosslinked polyacrylate polymers useful as thickeners or gelling agents including both cationic and nonionic polymers, with the cationics being generally preferred. Examples of useful crosslinked nonionic polyacrylate polymers and crosslinked cationic polyacrylate polymers are those described in U.S. Pat. No. 5,100,660, to Hawe et al, issued Mar. 31, 1992; U.S. Pat. No. 4,849,484, to Heard, issued Jul. 18, 1989; U.S. Pat. No. 4,835,206, to Farrar et al, issued May 30, 1989; U.S. Pat. No. 4,628,078 to Glover et al issued Dec. 9, 1986; U.S. Pat. No. 4,599,379 to Flesher et al issued Jul. 8, 1986; and EP 228,868, to Farrar et al, published Jul. 15, 1987.

### c) Polyacrylamide Polymers

The compositions of the present invention can optionally contain polyacrylamide polymers, especially nonionic polyacrylamide polymers including substituted branched or unbranched polymers. More preferred among these polyacrylamide polymers is the nonionic polymer given the CTFA designation polyacrylamide and isoparaffin and laureth-7, available under the Tradename Sepigel 305 from Seppic Corporation (Fairfield, N.J.).

Other polyacrylamide polymers useful herein include multi-block copolymers of acrylamides and substituted acrylamides with acrylic acids and substituted acrylic acids. Commercially available examples of these multi-block copolymers include Hypan SR150H, SS500V, SS500W, SSSA100H, from Lipo Chemicals, Inc., (Patterson, N.J.).

### d) Polysaccharides

A wide variety of polysaccharides are useful herein. "Polysaccharides" refer to gelling agents which contain a backbone of repeating sugar (i.e., carbohydrate) units. Nonlimiting examples of polysaccharide gelling agents include those selected from cellulose, carboxymethyl hydroxyethylcellulose, cellulose acetate propionate carboxylate, hydroxyethylcellulose, hydroxyethyl ethylcellulose, hydroxypropylcellulose, hydroxypropyl methylcellulose, methyl hydroxyethylcellulose, microcrystalline cellulose, sodium cellulose sulfate, and mixtures thereof. Also useful herein are the alkyl substituted celluloses. In these polymers, the hydroxy groups of the cellulose polymer is hydroxyalkylated (preferably hydroxyethylated or hydroxypropylated) to form a hydroxyalkylated cellulose which is then further modified with a C₁₀-C₃₀ straight chain or branched chain alkyl group through an ether linkage. Typically these polymers are ethers of C₁₀-C₃₀ straight or branched chain alcohols with hydroxyalkylcelluloses. Examples of alkyl groups useful herein include those selected from stearyl, isostearyl, lauryl, myristyl, cetyl, isocetyl, cocoyl (i.e. alkyl groups derived from the alcohols of coconut oil), palmityl, oleyl, linoleyl, linolenyl, ricinoleyl, behenyl, and mixtures thereof. Preferred among the alkyl hydroxyalkyl cellulose ethers is the material given the CTFA designation cetyl hydroxyethylcellulose, which is the ether of cetyl alcohol and hydroxyethylcellulose. This material is sold under the tradename Natrosol.RTM. CS Plus from Aqualon Corporation (Wilmington, Del.).

Other useful polysaccharides include scleroglucans which are a linear chain of (1-3) linked glucose units with a (1-6) linked glucose every three units, a commercially available example of which is Clearogel.TM. CS11 from Michel Mercier Products Inc. (Mountainside, N.J.).

### e) Gums

Other thickening and gelling agents useful herein include materials which are primarily derived from natural sources. Nonlimiting examples of these gelling agent gums include acacia, agar, algin, alginic acid, ammonium alginate, amylopectin, calcium alginate, calcium carrageenan, carnitine, carrageenan, dextrin, gelatin, gellan gum, guar gum, guar hydroxypropyltrimonium chloride, hectorite, hyaluroinic acid, hydrated silica, hydroxypropyl chitosan, hydroxypropyl guar, karaya gum, kelp, locust bean gum, natto gum, potassium alginate, potassium carrageenan, propylene glycol alginate, sclerotium gum, sodium carboyxmethyl dextran, sodium carrageenan, tragacanth gum, xanthan gum, and mixtures thereof.

Preferred compositions of the present invention include a thickening agent selected from carboxylic acid polymers, crosslinked polyacrylate polymers, polyacrylamide polymers, and mixtures thereof, more preferably selected from carboxylic acid polymers, polyacrylamide polymers, and mixtures thereof.

Preferably, the extract containing phenylethanoid glycosides is extracted from the dry stems of Cistanche tubulosa (Schenk.) Wight.

According to the invention, a process for preparing the extract containing phenylethanoid glycosides from Cistanche tubulosa (Schenk.) is provided. The process comprises the steps of:
a) Grinding the dry stem of cistanche tubulosa (Schenk.) Wight to form a crude powder;
b) Soaking the crude powder with water or ethanol, and refluxing the mixture at a temperature of 50-100°C and collecting the supernant;
c) Concentrating the supernatant obtained in step b) at a decreased pressure and centrifuging the concentrate;
d) Adding the centrifugate obtained in step c) onto a column which is packed with a macro-porous resin SEPABEADA;
e) Eluting the column with 10-60% of ethanol or deionized water;
f. Collecting the eluant and subjecting it to be dried under vacuum, crushed and sieved to obtain an extract containing phenylethanoid glycosides.

### The extraction of Cistanche tubulosa (Schenk.) Wight includes two procedures:

The primary extraction procedure includes soaking with water or ethanol, boiling, filtering, cooling, precipitating and centrifuging. The centrifugate obtained is the crude preparation. Preferably, the refluxing in step b) is repeated 2-4 times and the temperature is preferably from 70 to 90°C.

The refining procedure includes adding the centrifugate onto the column packed with a macro-porous resin, then adsorbing and eluting with a gradient concentration of ethanol or deionized water. The eluant obtained is a refining preparation, which is further dried under vacuum, crashed and sieved to obtain an extract containing phenylethanoid glycosides.

In combination with the above-mentioned carriers or adjuvants, the extract from Cistanche tubulosa (Schenk.) Wight can be formulated to obtain various cosmetics, such as facial mask, lotion, facial cream and ointment, and dermic preparations. These cosmetics and dermic preparations possess effects of overall skin improvement in softness, moisture, smoothness, whitening and spot reduction.

### Preparation of the Cosmetic Composition

The Cosmetic skin care compositions of the present invention are generally prepared by using conventional methods such as those known in the art. Such methods typically involve mixing of the ingredients in one or more steps to a relatively uniform state, with or without heating, cooling, application of vacuum, and the like.

### Methods for Regulating Skin Condition

The current Cosmetic compositions are useful in regulating mammalian skin. The preferred method for application is via leave-on products such as skin gels or skin creams. A less preferred method for application is via rinse-off or wipe-off products such as cleansers, both foaming cleansers and cold cream cleansers.

Regulating mammalian skin condition involves topically applying to the keratinous tissue a safe and effective amount of a composition of the present invention. The amount of the composition which is applied, the frequency of application and the period of use will vary widely depending upon the level of the skin care active substance and/or other components of a given composition and the level of regulation desired, e.g., in light of the level of keratinous tissue damage present or expected to occur.

In a preferred embodiment, the composition is chronically applied to the skin. By "chronic topical application" is meant continued topical application of the composition over an extended period during the subject's lifetime, preferably for a period of at least about one week, more preferably for a period of at least about one month, even more preferably for at least about three months, even more preferably for at least about six months, and more preferably still for at least about one year. While benefits are obtainable after various maximum periods of use (e.g., five, ten or twenty years), it is preferred that chronic application continue throughout the subject's lifetime. Typically applications would be on the order of about once per day over such extended periods, however application rates can vary from about once per week up to about three times per day or more.

A wide range of quantities of the compositions of the present invention can be employed to provide a skin appearance and/or feel benefit. Quantities of the present compositions which are typically applied per application are, in mg composition/cm² skin, from about 0.1 mg/cm² to about 10 mg/cm². A particularly useful application amount is about 1 mg/cm² to about 2 mg/cm².

Regulating mammalian skin tissue condition is preferably practiced by applying a composition in the form of a skin lotion, cream, gel, foam, ointment, paste, emulsion, spray, conditioner, tonic, cosmetic, lipstick, foundation, nail polish, after-shave, or the like which is preferably intended to be left on the skin or other keratin structure for some esthetic, prophylactic, therapeutic or other benefit (i.e., a "leave-on" composition). After applying the composition to the skin, it is preferably left on the skin for a period of at least about 15 minutes, more preferably at least about 30 minutes, even more preferably at least about 1 hour, still more preferably for at least several hours, e.g., up to about 12 hours. Any part of the external portion of the face, hair, and/or nails can be treated, e.g., face, lips, under-eye area, eyelids, scalp, neck, torso, arms, hands, legs, feet, fingernails, toenails, scalp hair, eyelashes, eyebrows, etc. The composition can be applied with the fingers or with an implement or device (e.g., pad, cotton ball, applicator pen, spray applicator, and the like).

Another approach to ensure a continuous exposure of the skin to at least a minimum level of the plant extract and skin care active is to apply the compound by use of a patch applied, e.g., to the face. Such an approach is particularly useful for problem skin areas needing more intensive treatment (e.g., facial crows feet area, frown lines, under eye area, and the like). The patch can be occlusive, semi-occlusive or non-occlusive and can be adhesive or non-adhesive. The composition can be contained within the patch or be applied to the skin prior to application of the patch. The patch can also include additional actives such as chemical initiators for exothermic reactions such as those described in U.S. Pat. Nos. 5,821,250, 5,981,547, and 5,972,957 to Wu, et al. The patch is preferably left on the skin for a period of at least about 5 minutes, more preferably at least about 10 minutes, more preferably still at least about 15 minutes, even more preferably at least about 20 minutes, still more preferably at night as a form of night therapy.

The benefits of the invention: Various skin care cosmetic compositions were prepared by blending an extract from Cistanche deserticola, especially Cistanche tubulosa (schenk.) Wight, as an essential active substance with other adjuvants and/or additives. No toxicity or side effect has been shown in acute and chronic toxicological experiments in animals, and no allergic response has been shown in clinical tests, either. In addition, the tyrosinase-inhibiting activity and the action on the capillary permeability of the cosmetic composition of the invention are several times of commercial freckle-removing cream. The finished products of the cosmetic composition of the invention show evident effects upon applying onto the human skin for several weeks. It has been shown in a series of tests that the extract from Cistanche tubulosa (schenk.) Wight possesses skin-whetening, freckle-removing and brightening effects, and has no toxicity or side effect to the skin. Even no stimulation has been shown in the skin. Thus, the cosmetic composition of the invention has a practical applicability in the industry.

### Preferred Embodiments

Preparation of the plant extract from cistanche tublosa (Schenk) wight:

### Example 1

10 kg of cistanche tublosa (schenk.) wight was crashed into a crude powder, then soaked in 50% ethanol in an amount which was 8 times of the crude powder. The mixture was heated at a temperature above 50°C for two hours. The mixture was filtered through a 100 mesh screen. The residue was then extracted twice with 50% ethanol in an amount which was 6 times of the residue, and the extraction was filtered through a 100 mesh screen. The combined filtrate was concentrated under vacuum (the degree of vacuum≥0.08Mpa) such that the concentrate has a specific gravity of 1.10. The concentrate was cooled and precipitated for 12 hours. The supernatant was centrifuged and the centrifugate was added onto a column packed with SEPABEADS resin. The column was eluted with two volume of 20% ethanol and the eluant was collected. The column was eluted twice, with two volume of 30% ethanol each time. The eluants of the total three times were combined and concentrated at a reduced pressure to obtain an extrative containing phenylethanoid glycosides and having a weight of 710 g.

The contents of acteoside and echinacoside were measured via a high performance liquid chromatography (HPLC), with the stationary phase being silicone of C18 alkyl silane; the mobile phase being acetonitrile-mathanol-1%acetic acid (10:15:75) aqueous solution in a flow rate of 1 ml/min, and the detecting wavelength being 334 nm.

Acteoside and echinacoside were weighed precisely and dissolved in the mobile phase to prepare reference solutions, with each 1 ml solution containing 0.1 mg ateoside and 0.14mg echinacoside, respectively.

The preparation of the test solution: 0.5g of extract was added in a 50ml brown measuring flask, into which 25ml of the mobile phase was added precisely. The flask was weighted precisely and kept standing for 0.5hr. After being ultrasonicated (230W, 35kHz, 40min), the flask was cooled and weighted again. The lost weight was made up with the mobile phase. Then, the flask was shaken, centrifuged and kept standing. The supernatant was removed to another brown bottle.

Measurement: The reference solution and the test solution were taken out in an amount of 2-10µl and 10-20µl , respectively, and injected into the liquid chromatography column. The peak areas of the acteoside and the echinacoside were measured. The contents were calculated from the peak areas. The content of the acteoside is 9.6 % while the content of the echinacoside is 5.2 % by weight of the preparation.

### Example 2

10 kg of cistanche tublosa (schenk.) wight was crashed into a crude powder, then soaked in 50% ethanol in an amount which was 8 times of the crude powder. The mixture was heated for two hours, then filtered through a 100 mesh screen. The residue was heated twice each with 50% ethanol in an amount which was 6 times of the residue, and the mixture was filtered through a 100 mesh screen. The combined filtrate was concentrated under vacuum (the degree of vacuum≥0.08Mpa) such that the concentrate has a specific gravity of 1.10. The concentrate was cooled and precipitated for 12 hours. The supernatant was centrifuged and the centrifugate was added onto a column packed with SEPABEADS resin. The column was eluted with two volume of 20% ethanol and the eluant was collected. Then the column was eluted with two volume of 30% ethanol. The later eluting step were repeated twice. The eluants of the total four times were combined and concentrated at a reduced pressure to obtain an extrative containing phenylethanoid glycosides and having a weight of 490 g. The content of the acteoside is 21.3 % while the content of the echinacoside is 9.2 % by weight of the preparation according to the above measurement.

### Example 3

10 kg of cistanche tublosa (schenk.) wight was crashed into a crude powder, then soaked in 50% ethanol in an amount which was 8 times of the crude powder. The mixture was heated for two hours, then filtered through a 100 mesh screen. The residue was heated three times each with 50% ethanol in an amount which was 6 times of the residue, and the mixture was filtered through a 100 mesh screen. The combined filtrate was concentrated under vacuum (the degree of vacuum≥0.08Mpa) such that the concentrate has a specific gravity of 1.10. The concentrate was cooled and precipitated for 12 hours. The supernatant was centrifuged and the centrifugate was added onto a column packed with SEPABEADS resin. The column was eluted with two volume of 30% ethanol and the eluant was collected. Then the column was eluted with two volume of 40% ethanol. The later eluting step were repeated three times. The eluants of the total five times were combined and concentrated at a reduced pressure to obtain an extrative containing phenylethanoid glycosides and having a weight of 160 g. The content of the acteoside is 65.3 % while the content of the echinacoside is 22.2 % by weight of the preparation according to the above measurement.

When the cistanche tublosa (schenk.) wight was extracted with water, the contents (wt%) of acteoside and echinacoside were higher than those when the extraction is undertaken with an alcohol.

Pharmacological effects: Skin melanocytes located in the basal layer of epidermis are the basis of the synthesis of melanine in the human pegmentary system. The melanine is synthesized from tyrosine enzymatically by tyrosinase in melanocytes and deposits in melanosomes to form uniform melamin granules gradually. The activity of the tyrosinase influnces directly the formation of the melanine. It has been shown in experiments that the inhibition of phenylethanoid glycosides on the activity of tyrosinase is significantly stronger than prunol, the well-known brightening agent used in the cosmetics (p>0.05).

It has been observed, in tissue culture experiments, that the cytolergy of M cells form a collagen fiber network, and acteoside, a phenylethanoid glycoside, possesses an obvious promotion on the growth of M cells, so as to accelerate the formation of the collagen fiber network. The difference in comparison with the contral group is statistically significant (P>0.05).

### Experimental Exmple

### 1. Acute toxicity (LD50, p.o.) experiment

Purpose: Observation on the adverse reaction and death condition resulted from the administration of test samples in a single dose, p.o., and determination of the half-lethal dose

### Basis of detection: GB 15193.3-1994

1.1 Materials and Method:
1.1.1 Sample: cistanche tublosa (schenk.) wight
1.1.2 Characters of the sample: Deep brown powder
1.1.3 Preparation of the sample: Sample 10000mg was weighted and grinded. Distilled water was added into the grinded sample to 20ml, and the mixture was blended sufficiently to form a uniform suspension which was used as a test sample.
1.1.4 Animal subject: 20 Kunming species of mice, 18-22g, half male and half female, provided by the experimental animal department, Fudan University. Certificate No.: 02-22-1. Temperature of bleeding room: 18-22°C. Relative humidity of bleeding room: 40-70%. Certificate No. of animal cabinet: 02-28. The feedstuffs were provided by Suhang Experimental Animal Tech-developing Co. and the Certificate No. of feedstuffs is Su E Shi Shen (2002) 006.
1.1.5 Instrumenttation: Electronic scale AC-3A 90401582, Equi-armbalance BP3100S - 91006909.
1.1.6 Test methods:
1.1.6.1 After fasting (no refraining from water) , the mice were selected based on the body weight, 10 male and 10 female, and housed into two cabinets, respectively. The difference of body weight is below 3g among the same sex mice.
1.1.6.2 Each animal was weighted and administrated with a single dose of the test sample in a volume of 0.4ml/20g via an intragastric catheter.
1.1.6.3 The general conditions, the change of weight, the toxic symptoms and the death conditions were observed for one week.
1.1.6.4 The animals were weighted again at the end of the experiment. The dead animals and the animals sacrificed at the end of the experiment were necrotomied and the pathologic changes were detected macroscopically.
1.1.6.5 The whole course and observed conditions were recorded in detail.
1.2 Results:

**Table 1 Results of acute toxicity test in mice (p.o.)**

| Sexuality | Dose | Animal | Death | Fatality |
|---|---|---|---|---|
| | (mg/kg) | (N) | (n) | ( % ) |
| Female | 10000 | 10 | 0 | 0 |
| Male | 10000 | 10 | 0 | 0 |

1.2.1 Cardinal symptoms : The activities of animals in each group are normal and the capills are in good color and glossiness during the whole experimental stage. No toxic symptom or death was observed.
1.2.2 Half-lethal dose: LD₅₀>10000mg/kg , both in female and male mice.
1.3 Conclusion : The test sample is substantially an asepsis substance according to the grading on the half-lethal dose.

### 2. Micronucleus Test:

### Purpose: Detection of marrow chromosomal aberration induced in mice in vivo Basis of detection: GB-15193.5-1994

### Sample: Cistanche tublosa (schenk.) wight

2.1 Characters of the sample: Extract from Cistanche tublosa (schenk.) wight, Deep brown powder
2.2 Preparation of sample: Sample 5000, 2500 or 1250mg was weighted and grinded. Distilled water was added into the grinded sample to 20ml, and the mixture was blended sufficiently to form a test sample.
2.3 Animal subject: Kunming species of mice, 25-30g, provided by experimental animal department, Fudan University. Certificate No.: 02-22-1.
2.4 Test condition: Temperature: 18-22°C. Relative humidity: 40-70%.
2.5 Test methods:
2.5.1 Animals were invided randomly into 5 groups, 10 in each group with half of female and half of male. In the five groups, there were three groups for three doses of test sample, one negative control group, distilled water, and one positive contral group, cyclophosphaide.
2.5.2. Animals were weighted and administrated with the test sample or contral substances in different concentrations in a volume of 0.4ml/20g via an intragastric catheter, and the administration is repeated once more in 30hr.
2.5.3 Animals were sacrificed via cervical bertebra dislocation 6 hr. after the second intragastric administration. The marrow was taken out of the femoral bone and blended uniformly with calf serum. Then, the film preparation, the fixation, and Giemasa stain were undertaken conventionally.
2.5.4 Test under microscope: Numbers of micronuclei were counted in over one thousand of philo-pleochromatic akaryocytes. Incidence rates of micronuclei were calculated and analyzed statistically.

2.6 Results :

**Table 2 Incidence rates of micronucle in philo-pleochromatic akaryocytes in animal marrow**

| Dose | Sex | No. | Cells tested | Micronucleated | Rate of | StatisticTest |
|---|---|---|---|---|---|---|
| (mg/kg) | | | | Cells No. | Micronuclei | (p value) |
| | | | | | (%o) | |
| 5000 | M | 5 | 5000 | 4 | 0.8 | >0.05 |
| 5000 | F | 5 | 5000 | 3 | 0.6 | >0.05 |
| 2500 | M | 5 | 5000 | 5 | 1.0 | >0.05 |
| 2500 | F | 5 | 5000 | 3 | 0.6 | >0.05 |
| 1250 | M | 5 | 5000 | 4 | 0.8 | >0.05 |
| 1250 | F | 5 | 5000 | 4 | 0.8 | >0.05 |
| Dis.water | M | 5 | 5000 | 4 | 0.8 | |
| (20000mg/kg) | F | 5 | 5000 | 4 | 0.8 | |
| Cyclophosphaide | M | 5 | 5000 | 132 | 26.4 | <0.01 |
| (40mg/kg) | F | 5 | 5000 | 129 | 25.8 | <0.01 |

| | | | | | | |
|---|---|---|---|---|---|---|
| * Compared with the negative contral group ( chi square test) . | | | | | | |

2.7 Conclusion : Negative results of the Extract from Cistanche tublosa (schenk.) wight.have been shown statistically in the micronucleus test of eosinophil cells in marrow.

### 3. Teratospermia Test:

### Purpose: Detection of the influence of the test sample on the gonepoiesis and the development of spermatozoa, and detection of the genetoxic on the cell granule in vivo Basis of detection: GB-15193.7-1994

3.1 Charaters of the sample: Extract from Cistanche tublosa (schenk.) wight, Deep brown powder
3.2 Preparation of sample: Sample 5000, 2500 or 1250mg was weighted and grinded. Distilled water was added into the grinded sample to 20ml, and the mixture was blended sufficiently to obtain a test sample.
3.3 Animal subject: Male mice, depuratory grade, 25-30g, provided by experimental animal department, Fudan University. Certificate No.: 02-22-1.
3.4 Test condition: Temperature: 18-22□. Relative humidity: 40-70%.
3.5 Test methods:
3.5.1 Animals were invided randomly into 5 groups, 5 in each group. It the five groups, there were three groups for three doses of test sample, one negative contral group, distilled water, and one positive contral group, cyclophosphaide.
3.5.2. Animals were weighted and administrated with the test sample or contral substances in different concentrations in a volume of 0.4ml/20g via an intragastric catheter, once a day for 5 days.
3.5.3 Animals were sacrificed via cervical bertebra dislocation 35 days after the first intragastric administration. The ambo-epididymises were taken off and put into normal saline. After being cut into pieces, they were filtered through a four layer gauze. The filter was smeared, fixed and dyed with 2% eosin.
3.5.4 Test under micscope: Amounts of teratosperm in 1000 spernatozoas were counted per mouse and rates of teratospermia were calculated statistically.
3.6 Results : 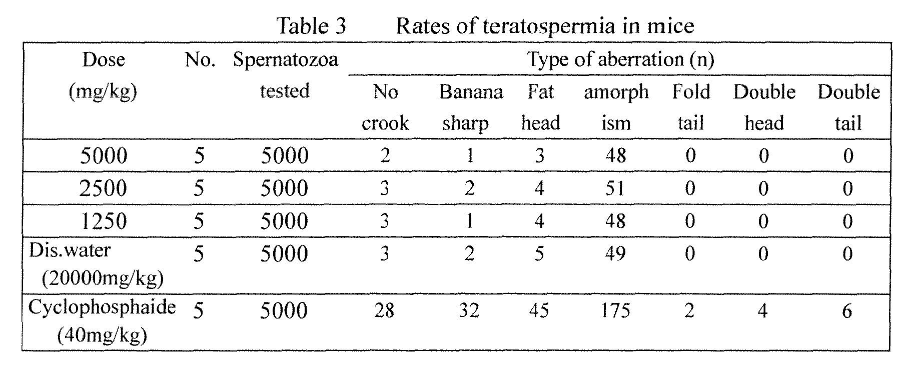 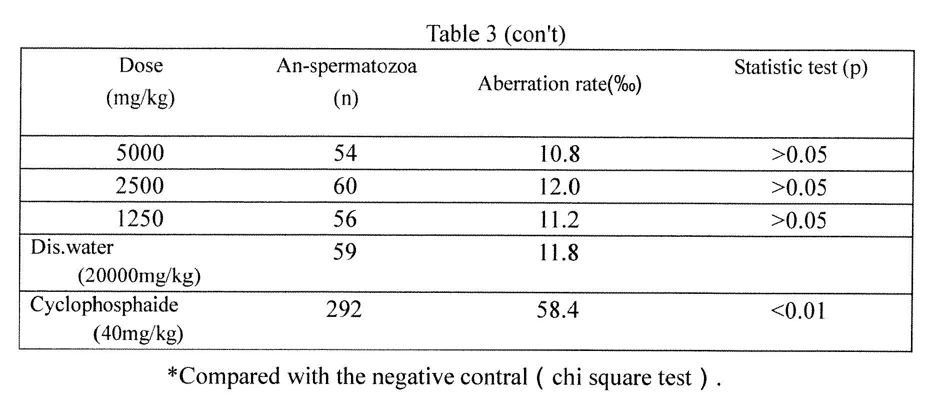
3.7 Results : Negative results have been shown statistically in the teratospermia test of the Extract from Cistanche tublosa (schenk.) wight.

### 4, AmesTest :

Purpose :Detection of Mutagenicity of the test substance to predict its genetic injury and potential carcinogenesis
Basis ofdetection : GB15193.4 - 1994

4.1 Charaters of the sample: Extract from Cistanche tublosa (schenk.) wight, Deep brown powder
4.2 Solvent : Sterile distilled water
4.3 Sample processing and preparation : 4000mg sample was weighted and distilled water was added into the sample to 20ml, then the mixture was blended to obtain a 200mg/ml of solution. 3 , 1 , 0.3 and 0.1mL of this solution were taken, respectively and each was added with sterile distilled water to 10ml to obtain a solution in the concentration of 60mg/ml, 20mg/ml, 6mg/ml and 2mg/ml, respectively.
4.4 Test doses : 0.2mg/dish, 0.6mg/dish, 2mg/dish, 6mg/dish and 20mg/dish
4.5 Environic condition of the experiment : Temperature : 18-22°C , Relative humidity : 50 - 70%
4.6 Instrumenttation : Incubator PYX-DHS 193
Thermostatic water bath DK-600
Electronic balance AE163 1010002
4.7 Test strains : TA97, TA98, TA100 and TA102, with qualitied biological charaters, provided by Biochemistry Dept., California University, CA, America. The concentration of the bacteria liquid used for the test was 1-2× 10⁹/ml.
4.8 Induction and preparation of rat liver S₉ :
A healthy adult SD rat with a body weight of about 150g was selected. Polychlorinatedbiphenyl was dissolved into corn oil to obtain a solution of 200mg/ml. The above solution was injected (i.p.) , in a sterile operation, into the rat in a dose of 500mg/kg. 5 days later, the animal was sacrificed via decapitation. The liver was taken out and weighted, then rinsed several times with 0.15mol/L of fresh cooled solution of potassium chloride. The amount of the potassium chloride solution was 3ml/g liver ( wet weight ). The beaker in which the rinsed liver put was removed into an ice bath and the liver was cut into pieces with a pair of sterilized scissors. The liver was homogenized with a tissue homogenizer( 2000r/min , 1min ) , then the liver homogenate thus prepared was centrifuged in a high speed centrifuge (0-4°C, 9000g, 10min). The supernatant, S₉ part , was packed into several sterile tubes and conserved in liquid nitrogen. The above steps need to be operated in a sterile and cooled local circumstance. The S₉ thus prepared was further identified for its content of protein and bioactivity of indirect mutagen. Result of identification : the sterility test was qualified, the content of protein was 33mg/ml, and the S₉ bioactivity conformed with the criterion (0.5ml S₉ mixture/dish, in which 50µl of S₉ was contained ) .
4.9 Solvent contral : Sterilee distilled water
4.10 Positive contral :
-S₉ : TA97 Atebrin ( 500µg/dish ) , TA98 p-nitroquinoline ( 200µg/dish ) ,
TA100 ( 1µ/dish ) , TA102 ( 1µl/dish ) , and MMS ( 1µ/dish ).
+ S₉ :TA97, TA98, TA100 2-aminofluorene( 10µg/dish ) ,TA102 1,8-dioxy anthraquinone ( 50µl/dish ) .
The above positive contral substances were dissolved in dimethyl sulfoxide except Atebrin which was dissolved in sterile distilled water.
4.11 Method of test :
Standard plate incorporation on the basis of GB15193 - 94 : Into the top agar (45°C, 2ml) ,the bacterial liquid 0.1ml, Sample 0.1ml and S9 mixture 0.5ml for activation were added sequently. The mixture was blended sufficiently and poured rapidly onto the underlayer medium. After the incubation of 48 hr. at 37□ , results were observed. each test was repeated once more.
4.12 Results of the first test :

**Table 4**

| Dose (mg/dish) | Re-established colonies ( colonies/sish ) *X̅* ±SD | | | | | | | |
|---|---|---|---|---|---|---|---|---|
| | TA97 | | TA98 | | TA100 | | TA102 | |
| | -S₉ | + S₉ | -S₉ | + S₉ | -S₉ | + S₉ | -S₉ | + S₉ |
| 0.2 | 147±7 | 154±3 | 35±4 | 37±5 | 162±8 | 164±6 | 246±10 | 249±7 |
| 0.6 | 150±5 | 148±6 | 36±2 | 35±3 | 164±6 | 161±4 | 244±8 | 251±11 |
| 2.0 | 153±4 | 150±4 | 34±5 | 36±4 | 158±7 | 163±7 | 251±8 | 246±7 |
| 6.0 | 146±6 | 147±8 | 37±3 | 35±3 | 165±6 | 159±7 | 243±7 | 245±9 |
| 20.0 | 141±5 | 148±8 | 36±4 | 38±2 | 157±5 | 163±8 | 236±7 | 240±10 |
| Blank | 148±6 | 154±4 | 36±3 | 37±5 | 163±7 | 158±8 | 247±10 | 250±6 |
| Solvent | 151±7 | 148±5 | 35±4 | 34±4 | 161±6 | 165±4 | 245±8 | 246±7 |
| Atebrin (500µg/dish) | 1626±109 | | | | | | | |
| p-nitroquinoline ( 200µg/dish ) | | | 1108±73 | | | | | |
| Methyl mesylate(1µl/dish ) | | | | | 1463±84 | | 2784±289 | |
| 2-aminofluorene ( 10µg/dish ) | | 1727±64 | | 2196±143 | | 1618±118 | | |
| 1,8-dioxy anthraquinone ( 50µl/dish ) | | | | | | | | 885±56 |

4.13 Results of the second test :

**Table 5**

| Dose (mg/dish) | Re-established colonies ( colonies/sish ) *X̅* ±SD | | | | | | | |
|---|---|---|---|---|---|---|---|---|
| | TA97 | | TA98 | | TA100 | | TA102 | |
| | -S₉ | + S₉ | -S₉ | + S₉ | -S₉ | + S₉ | -S₉ | + S₉ |
| 0.2 | 139±5 | 142±7 | 40±5 | 41±3 | 146±7 | 149±8 | 267±8 | 260±9 |
| 0.6 | 136±6 | 145±6 | 39±3 | 40±3 | 150±6 | 151±4 | 264±7 | 266±1 |
| 2.0 | 142±4 | 145±7 | 38±3 | 38±5 | 148+4 | 149±7 | 267±10 | 246±6 |
| 6.0 | 137±8 | 138±8 | 41±2 | 41±6 | 145±5 | 143±6 | 258±4 | 261+9 |
| 20.0 | 135±6 | 140±6 | 37±4 | 38±3 | 142±5 | 150±5 | 259±8 | 256±6 |
| Blank | 136±6 | 144±5 | 39±5 | 40±4 | 147±6 | 149±3 | 264±9 | 270±7 |
| Solvent | 143±6 | 41±3 | 43±3 | 144±7 | 146±5 | 267±6 | 268±4 | 143±6 |
| Atebrin (500µg/dish) | 1478±47 | | | | | | | |
| p-nitroquinoline ( 200µg/dish ) | | | 868±60 | | | | | |
| Methyl mesylate(1µl/dish ) | | | | | 1641±80 | | 2520±112 | |
| 2-aminofluorene ( 10µg/dish ) | | 1772±137 | | 2774±164 | | 1548±124 | | |
| 1,8-dioxy anthraquinone ( 50µl/dish ) | | | | | | | | 975±86 |

4.14 Results : Negative results have been shown in the Extract from Cistanche tublosa (schenk.) wight. in the above test condition.

### 5, 30 day Feeding trial

Purpose : Further detection on the toxic action of the sample and estimation of the maximum non-toxic dose
Basis of detection : GB15193.7 - 1994

5.1 Name of the sample : Cistanche tublosa (schenk.) weight
5.2 Charaters of the sample: Deep brown powder
5.3 Design of doses : The recommended dose for human body is 1080mg/60kg/d. There were three dose groups, 180 mg/kg (high), 900 mg/kg (middle) and 1800 mg/kg (low) , corresponding to 10 times , 50 times and 100 times of the recommended dose for human body , respectively, and a blank contral group.
5.4 Sample processing :
According to the dose design of the test and the feed intaken by the animal (about 10g/100g BW/d ) , 18, 90 and 180g of sample were incorporated into 10kg of feed, respectively, mixed thoroughly, and granula feedstuffs containing high, middle or low amount of sample were produced. Three groups of animals were feeded with these three dose feedstuffs. The blank contral group of animals were feeded with the similar granula feedstuffs except having no sample.
5.5 Experimental animals and the circumstance :
5.5.1 80 SD rats , 60-80g, half male and half female, provided by experimental animal department, Fudan University. Certificate No.: 02-22-2.
5.5.2 Temperature of bleeding: 18-22□. Relative humidity: 40-70%. Certificate No. of animal cabinet: 02-28. The feedstuffs were provided by Suhang Experimental Animal Tech-developing Co. and the Certificate No. of feedstuffs is Su E Shi Shen (2002) 006.
5.6 Instrumenttation:
Automatic biochemical equipment AL800 (Shimadzu, Japan) 15R325
Blood analyser CD3700 (Cell-DYN America Corporation) 15R325
Desk centrifuge Mikro22 ( HETTTCH Company) 1105
Eletronic balance Bp3100s ( Sand Sartorius Corporation ) 91006823
Eletronic balance JA1003 ( Shanghai Balance Factory ) 300
Eletronic scale ACS-3 ( Weighing Apparatus Factory, Shanghai Wo ) 1050480
Eletronic scale ACS-3 (Weighing Apparatus Factory, Shanghai Wo ) 90401570

5.7 Method of test :
5.7.1 Animals were divided randomly into four groups , 20 for each group , half male and half female , three groups for three doses, respectively, and one group for the blank contral. The animals were kept alone , with food and drink, ad lib., for 30 days.
5.7.2 The body weights were weighed and the intake of the foodstuffs were recorded at the beginning of the test and once a week in the whole period of test.
5.7.3 30 Days latter, rats were weighed and blood samples were collected for the hematologic and biochemical analysis. Then rats were sacrificed, dissected and observed macroscopically to detect any obvious pathological changes. The liver, kidney, spleen and sex organs, etc., were weighed for the calculation of the ratio of organ weight to body weight, and the liver, kidney, spleen, gastro-intestine and sex organs, etc. were histopathologically inspected
Results :
5.8.1 Growth status and food availiability :

**Table 6 Growth of body weight of experimental animals (g) ( X̅ ±SD )**

| Sex | Group | 0 week | 1 week | 2 weeks | 3 weeks | 4 weeks |
|---|---|---|---|---|---|---|
| ♂ | Control | 71±6 | 122±10 | 177±12 | 228±15 | 279±19 |
| | Low-dose | 70±6 | 120±10 | 175±12 | 224±17 | 282±23 |
| | Intermindiat-dose | 71±7 | 122±10 | 178±13 | 226±18 | 284±24 |
| | High-dosage | 71±6 | 119±9 | 178±14 | 227±19 | 276±24 |
| ♀ | Control | 71±7 | 113±9 | 150±11 | 171±13 | 292±13 |
| | Low-dose | 71±7 | 114±9 | 148±10 | 171±12 | 188±17 |
| | Intermindiat-dose | 70±7 | 114±9 | 147±11 | 169±12 | 186±16 |
| | High-dosage | 72±6 | 114±8 | 148±11 | 170±13 | 184±15 |

**Table 7 Food availiability of experimental animals ( X̅ ±SD )**

| Sex | Group | Beginning(g) | End(g) | Growth(g) | Ingestion(g) | Food availiability(%) |
|---|---|---|---|---|---|---|
| ♂ | Control | 71±6 | 279±19 | 208±15 | 643±39 | 32.3±1.2 |
| | Low dose | 70±6 | 282±23 | 212±17 | 632±49 | 33.6±1.8 |
| | Intermindiat dose | 71±7 | 284±24 | 213±17 | 673±43 | 31.6±1.0 |
| | High dosage | 71±6 | 276±24 | 205±19 | 632±68 | 32.5±1.5 |
| ♀ | Control | 71±7 | 192±13 | 121±9 | 524±49 | 23.3±1.7 |
| | Low dose | 71±7 | 188±17 | 117±12 | 512±54 | 22.9±1.7 |
| | Intermindiat dose | 70±7 | 186±16 | 116±11 | 530±57 | 21.8±1.4 |
| | High dosage | 72±6 | 184±15 | 112±10 | 498±42 | 22.7±1.9 |

It is shown in the above two tables that the grouth of rats in each sample group is essentially fine.

**Table 8 Gross anotomy and macroscopic observation in kidney**

| Group | | | *High dose | Blank |
|---|---|---|---|---|
| Animal | | | 20 | 20 |
| Cortical part | Renal capsule changing(cases) | | 0 | 0 |
| | Renal tubular epithelial cell denaturalization | Necrosis(cases) | 0 | 0 |
| | | Edema(cases) | 0 | 0 |
| | | Vacuolated changes(hydropic degeneration and fatty degeneration (cases) | 0 | 0 |
| | | Cast nephropathy(cases) | 4 | 5 |
| | | Inflammatory cell infiltration(cases) | 0 | 0 |
| | Glomerular denaturalization(cases) | | 0 | 0 |
| | Tubulointerstitial lesion(inflammatory cell infiltration) (cases) | | 0 | 0 |
| | Others(cases) | | 0 | 0 |
| Renal medulla | Renal tubule denaturalization | Necrosis(cases) | 0 | 0 |
| | | Edema(cases) | 0 | 0 |
| | | Vacuolated changes(hydropic degeneration and fatty degeneration (cases) | 1 | 0 |
| | | Cast nephropathy(cases) | 0 | 0 |
| | | Inflammatory cell infiltration(cases) | 0 | 0 |
| | | Tubulointerstitial inflammatory cell infiltration (cases) | 0 | 0 |
| | | Others(cases) | 0 | 0 |
| | Renal pelvic papillary changes(cases) | | 0 | 0 |
| | Transitional epithelium changes(cases) | | 0 | 0 |

| | | | | |
|---|---|---|---|---|
| * It has been shown, in the gross anotomy and macroscopic observation, that the kidneys of rat in each sample group have no obvious pathological change , thus only rats in high dose group and contral group were histologically inspected. | | | | |

**Table 9 Gross anotomy and macroscopic observation in spleen**

| | Blank | | *High dose | |
|---|---|---|---|---|
| | Female | Male | Female | Male |
| Animal (N) | 10 | 10 | 10 | 10 |
| Red pulp extention(cases) | 0 | 0 | 0 | 0 |
| White pulp atrophy(cases) | 0 | 0 | 0 | 0 |
| Hematopoietic cell hyperplasia(cases) | 0 | 0 | 0 | 0 |
| Inflammatory cells infiltrate(cases) | 0 | 0 | 0 | 0 |
| Hyperpigmentation(cases) | 0 | 0 | 0 | 0 |
| Others(cases) | 0 | 0 | 0 | 0 |

| | | | | |
|---|---|---|---|---|
| * It has been shown, in the gross anotomy and macroscopic observation, that the spleens of rat in each sample group have no obvious pathological change , thus only rats in high dose group and contral group were histologically inspected. | | | | |

**Table 10 Gross anotomy and macroscopic observation in gastro-intestine**

| | Blank | | *High dose | |
|---|---|---|---|---|
| | Female | Male | Female | Male |
| Animal (N) | 10 | 10 | 10 | 10 |
| Mucosal bleeding (cases) | 0 | 0 | 0 | 0 |
| Edema(cases) | 0 | 0 | 0 | 0 |
| Inflammatory cells infiltrate(cases) | 0 | 0 | 1 | 0 |
| Necrosis(cases) | 0 | 0 | 0 | 0 |
| Atrophy (cases) | 0 | 0 | 0 | 0 |
| Proliferation (cases) | 0 | 0 | 0 | 0 |

| | | | | |
|---|---|---|---|---|
| * It has been shown, in the gross anotomy and macroscopic observation, that the gastro-intestine of rat in each sample group has no obvious pathological change , thus only rats in high dose group and contral group were histologically inspected. | | | | |

**Table 11 Gross anotomy and macroscopic observation in didymus**

| | Blank | *High dose |
|---|---|---|
| Animal (N) | 10 | 10 |
| Atrophy(cases) | 0 | 0 |
| Denaturalization (cases) | 0 | 0 |
| Bleeding(cases) | 0 | 0 |
| Edema (cases) | 0 | 0 |
| Cysts(cases) | 0 | 1 |
| Others(cases) | 0 | 0 |

| | | |
|---|---|---|
| * It has been shown, in the gross anotomy and macroscopic observation, that the didymus of rat in each sample group has no obvious pathological change , thus only rats in high dose group and contral group were histologically inspected. | | |

**Table 12 Gross anotomy and macroscopic observation in cvaries**

| | Blank | *High dose |
|---|---|---|
| Animal (N) | 10 | 10 |
| Atrophy(cases) | 0 | 0 |
| Denaturalization (cases) | 0 | 0 |
| Bleeding(cases) | 0 | 0 |
| Edema (cases) | 0 | 0 |
| Cysts(cases) | 2 | 1 |
| Others(cases) | 0 | 0 |

| | | |
|---|---|---|
| * It has been shown, in the gross anotomy and macroscopic observation, that Ovaries of rat in each sample group have no obvious pathological change , thus only rats in high dose group and contral group were histologically inspected. | | |

The sample, the extract from Cistanche tublosa (schenk.) wight, was toxicological evaluated according to the stipulations in GB 15193 - 1994, the results are as follows [( Inspec. No. 5970B, Tox. ( 2003 ) 0034 ) , Preventive Medicine Research Center, Shanghai ( 2003 )] :
1. Acute toxicity test: LD₅₀ (p.o.) > 10000mg/kg, both in female and male mice. The test sample is substantially an asepsis substance according to the grading on the half-lethal dose.
2. Micronucleus Test: negative results.
3. Teratospermia Test: negative results.
4. AmesTest: negative results.
5. 30 Day Feeding Trial: Aminals in each group were well grown. There is no obvious difference between the sample group and the contral group in the results of hematologic analysis, biochemical analysis, ratio of main organ weight to body weight and histologic inspection.
6. Stimulation Test:
1 ) 6 Guinea pigs , 250±10g , half male and half female, the skin was shaved on two sides of vertebra 24hr. prior to the administration of sample , and the shaved area was 8x5cm². The skin was inspected 24hr. after shaving to ensure that no skin destruction exists.
   Subject contrast was adopted on the left side and right side. The left shaved side was applied with 1g of the sample containing 2% of the extract from Cistanche tublosa (schenk.) wight and other cosmetic adjuvants , and the right side was applied with 1 g of excipients as a contral. The skin of each side was covered with a film, gauzes and fixed with adhesive tapes. The guinea pigs were kept alone. 24hr. later, the test sample was washed-out with warm water. Then the areas of skin were macroscopically observed and histopathologically inspected 1hr., 24hr., 48hr. and 72hr., respectively, after the washing-out of the test sample. Also, the conditions of the position applied with the test sample were recorded and scored, such as erythema and/or oedema, if any. The scores and the recovery of above conditions were recorded every day. The average scores are listed in the following table for the evaluation of the stimulation of the test sample on the integrated skin (Table 13). 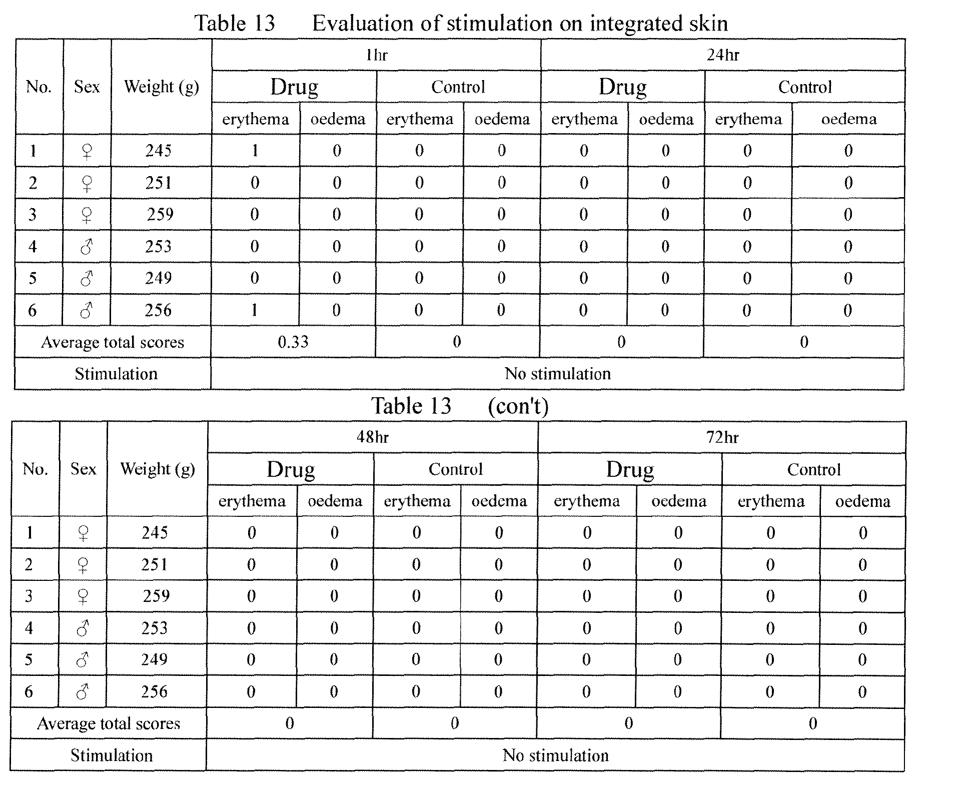
2 ) 6 Guinea pigs , 250±10g , half male and half female, the skin was shaved on two sides of vertebra 24hr. prior to the administration of sample , and the shaved area was 8 x 5cm². The skin was sterilized and scarificated with a Scalpel to form a "#" scarification in a depth such that capillary hemorrhage can be seen. The damaged degree of skin is identical essentially on both sides.

Subject contrast was adopted on the left side and right side. The left shaved side was applied with 1g of the sample containing 2% of the extract from Cistanche tublosa (schenk.) wight and other cosmetic adjuvants , and the right side was applied with 1g of excipients as a contral. The skin of each side was covered with a film, gauzes and fixed with adhesive tapes. The guinea pigs were kept alone. 24hr. later, the test sample was washed-out with warm water. Then the skins were macroscopically observed and histopathologically inspected 1hr., 24hr., 48hr. and 72hr., respectively, after the washing-out of the test sample. Also, the conditions of the locations applied with the test sample were recorded and scored, such as erythema and/or oedema, if any. The scores and the recovery of above conditions were recorded every day. The average scores are listed in the following table 11 for the evaluation of the stimulation of the test sample on the damaged skin (Table 14). 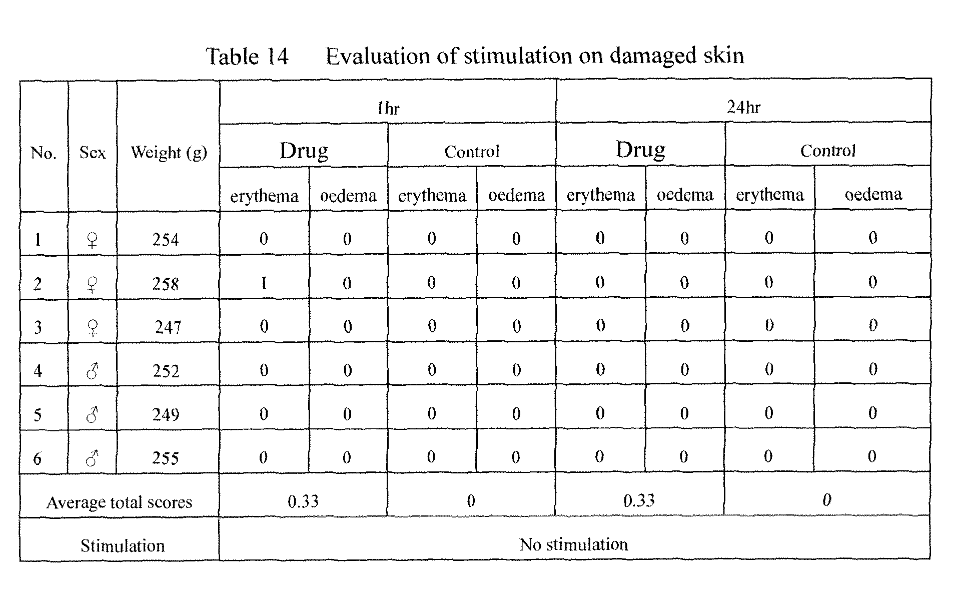 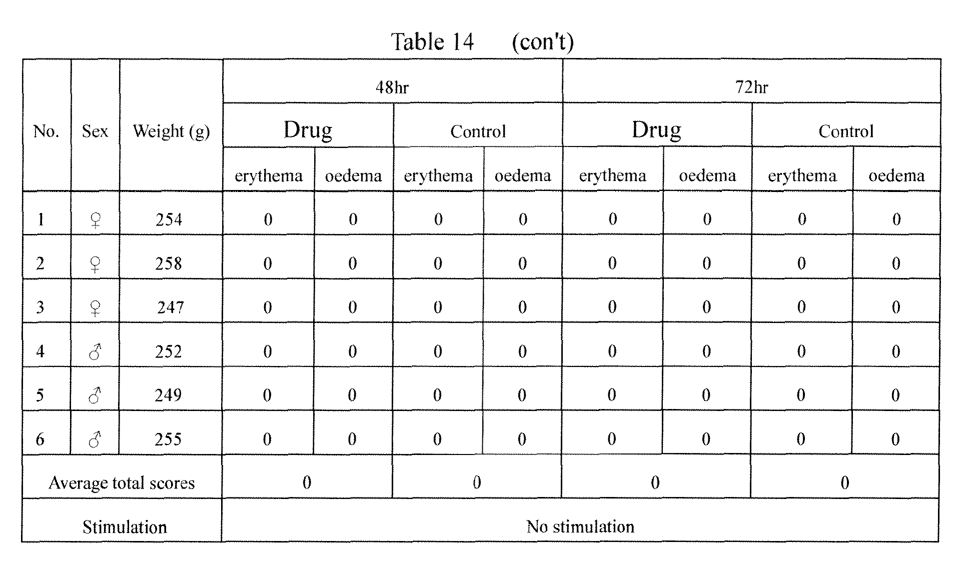
7. Capillary permeability test
42 Mice were divided randomly into two groups, i.e., the experimental group, the cosmetic comosition of the invention containing 2% of the extract from Cistanche tublosa (schenk.) wight and other cosmetic adjuvants; and the contral group, the commercially available freckle cream containing arbutin as a main active. The abdomen of each mouse was unhaired with a depilat. 36hr. later, the cosmetic comosition of the invention or the commercially available freckle cream was applied on the abdomen of each mouse, 0.25g/10g, twice, with an interval of 20 min. Then, 0.02ml of histamine solution was injected intracutaneously on the abdomen , at 1-2 position(s) per mouse. 0.2ml of 1% Evans Blue solution was injected from the vena caudalis during 1-2 min. The mice were sacrificed via cervical vertebra luxatio 15min.later. The abdomen skin was splited and the blued area was measured with a caliper rule.

**Table 15**

| Group | Animal (N) | Blued area ( mm² ) |
|---|---|---|
| Cosmetic comosition of the invention | 21 | 87.3±8.2 |
| Commercially available freckle cream | 21 | 56.2±4.9 |

8. Inhibition on tyrosinase
Materials: Tyrosinase available from Sigma; 3,4-dihydroxyphenylalanine Content>98% , BR (Shanghai Chemical Agent Factory); the cosmetic coposition of the invention; and the commercially available freckle cream.
Experimental method : The cosmetic comosition of the invention containing 2% of the extract from Cistanche tublosa (schenk.) wight and other cosmetic adjuvants and the commercially available freckle cream containing arbutin as a main active, both in the form of 50% methanol extract, were suspensed in a phosphate buffer (pH 6.8). This suspensions were diluted with the buffer to desired concentrations for use.
Determination of the enzyme activity : According to the Method described by Mason & Nagatsu , a mixture of 0.1ml tyrosinase solution (1.375µl), 0.9ml phosphate buffer and 1ml buffer containing or not containing the extract from Cistanche tublosa (schenk.) wight was incubated (25°C, 10min), then 1ml of 0.03% 3,4-Dihydroxyphenylalanine was added and the mixture was incubated (25°C, 2min). The incubation mixture was determined in 475nm to obtain the absorbance, then the inhibition percentage on the tyrosinase was calculated. 50% Inhibition concentration (ID₅₀) was used to evaluate the inhibition of the extract from Cistanche tublosa (schenk.) wight on the tyrosinase (Table 16).

**Table 16**

| | Concentration | Dose | Tyrosinase | Inhibition ratio |
|---|---|---|---|---|
| Cosmetic composition of the invention | 2.9mg/ml | 1ml | 1ml | 72.1% |
| Commercially available freckle cream | 2.9mg/ml | 1ml | 1ml | 16.5% |

9. Application on human body
Subjects :
The cosmetic composition made from 2% of the extract from Cistanche tublosa (schenk.) wight, as an active component, was applied to 60 women, 18-65 years old , to observe the freckle-removing and whitening effects. The applied sites were those with locus niger, colored spot, age pigmen, moth-patch, aestates, macula solaris, butterfly spot and cyasma, etc.
Application method : The cosmetic composition was applied uniformly on the facial skin and left dorsum of hand without a time limit for the remaining, 2-4 times per day, for 2-4 weeks.
Judgment on effects :
Obvious amelioration :Colored spots formed due to the accrescence of skin pigment resulting from various factors almost disappear or may only be seen indistinctly; the skin of dorsum of the left hand was obvious pure white, exquisite, slick and flexible in comparison with the skin of dorsum of the right hand.
Amelioration : Various colored spots were lightened , more than 30% of colored spots faded away , the skin of dorsum of the left hand was white and exquisite comparing with that of the right hand.
Inefficacy: No change in colored spots , the skin of dorsum of both hands were in an equal color.
Results: There were 16 obvious amelioration cases; 35 amelioration cases; and 9 inefficacy cases. The total effective rate was 85%.

It is shown that effective rate of the cosmetic composition of the invention is 85% and the effects are obvious within 2-4 weekd after the conventional application in a normal dose.

### Preparation of the cosmetic composition of the invention

### Examples 1 ∼ 6

The following topical skin care compositions containg the plant extract from cistanche tublosa (Schenk) wight. The process for the preparation of these compositions are similar to those used for the preparation of common cosmetic composition. The weight percents of acteoside and echinacoside in the extract from cistanche tublosa (Schenk) wight are 9.6% and 7.2%, 27.1% and 11.0%, 71.3% and 8.2%, 37.9% and 23.5%, 8.7% and 5.2%, and 75.1% and 12.2%, respectively, in Formula 1-6.

**Table 17**

| Formula | 1 | 2 | 3 | 4 | 5 | 6 |
|---|---|---|---|---|---|---|
| Extract from cistanche tublosa (Schenk) wight | 2.0 | 1.0 | 0.5 | 1.0 | 2.0 | 2.0 |
| Deionized water | 71.65 | 65.45 | 63.7 | 61.25 | 61.0 | 58.34 |
| Xanthan Gum | 0.3 | 0 | 0 | 0 | 0 | 0 |
| Glycerin | 5.0 | 0 | 0 | 12.0 | 12.0 | 12.0 |
| Propylene Glycol | 3.0 | 0 | 0 | 0 | 0 | 0 |
| Disodium EDTA | 0.2 | 0 | 0 | 0.2 | 0.2 | 0.2 |
| Carbopol #934 | 0.3 | 0.2 | 0.2 | 0 | 0 | 0 |
| Isopropyl Palmitate | 2.4 | 0 | 2.0 | 0 | 0 | 0 |
| Cetyl Alcohol | 1.5 | 3.0 | 3.0 | 0.55 | 0.6 | 0.55 |
| Ethylhexyl palmitate | 4.0 | 0 | 0 | 0 | 0 | 0 |
| Methyl paraben | 0.4 | 0.3 | 0.3 | 0.2 | 0.3 | 0.2 |
| Tocopheryl acetate | 0.1 | 0 | 0.1 | 0 | 0 | 0 |
| Vitamin A palmitate | 0.1 | 0 | 0.1 | 0 | 0 | 0 |
| Petrolatum | 2.0 | 6.0 | 5.0 | 0 | 0 | 0 |
| Ethylene glycol distearate | 0.5 | 1.5 | 1.0 | 0 | 0 | 0 |
| Stearyl alcohol ethoxylated (20-40) | 2.7 | 1.25 | 2.0 | 0 | 0 | 0 |
| Triethanotamine | 0.3 | 0.2 | 0.2 | 0 | 0 | 0 |
| Cyclomethicone D5 | 2.0 | 3.0 | 2.0 | 0 | 0 | 0 |
| Cyclomethicone Blend | 1.0 | 1.0 | 2.0 | 0 | 0 | 0 |
| Germall | 0.5 | 0 | 0 | 0 | 0 | 0 |
| D&C Yellow 10 | 0.05 | 0.05 | 0.05 | 0 | 0 | 0 |
| Hydroxyethyl cellulose | 0 | 0.3 | 0.3 | 0 | 0 | 0 |
| Mineral oil | 0 | 14.3 | 15.0 | 0 | 0 | 0 |
| Myristyl myristate | 0 | 2.0 | 2.0 | 0 | 0 | 0 |
| Butylated hydroxytoluene | 0 | 0.05 | 0.06 | 0 | 0 | 0 |
| Imidazalidinyl urea | 0 | 0.3 | 0.5 | 0 | 0 | 0 |
| Almond oil | 0 | 0.1 | 0 | 0 | 0 | 0 |
| Potassium hydroxide 45% | 0 | 0 | 0 | 2.42 | 2.5 | 2.4 |
| Stearic acid | 0 | 0 | 0 | 18.0 | 15.0 | 18.0 |
| Silicone fluid SF-96 | 0 | 0 | 0 | 2.0 | 2.0 | 2.0 |
| Ethylhexyl | 0 | 0 | 0 | 1.25 | 1.25 | 1.25 |
| methoxycinnamate | | | | | | |
| Benzyl alcohol | 0 | 0 | 0 | 0.5 | 0.5 | 0.5 |
| Licorice Extract PT-40 | 0 | 0 | 0 | 0.1 | 0.1 | 0 |
| Butylene glycol | 0 | 0 | 0 | 0.5 | 0.5 | 0.5 |
| Color and dye | 0 | 0 | 0 | 0.065 | 0.05 | 0.06 |
| Ascorbyl glucoside | 0 | 0 | 0 | 0 | 2.0 | 0 |
| Titanium dioxide | | | | | | 1.0 |
| Zinc oxide | | | | | | 1.0 |

It is shown, from the above experiment Example, that the cosmetic composition of the invention possesses freckle-removing, skin-whitening and brightening effects, which are many times better than the congener products available commercially. The cosmetic composition of the invention has no toxicity or side effect to human body and skin.

## Claims

**1.** A cosmetic composition, comprising an effective amount of Genus cistanche extract and other useful cosmetic adjuvant(s).

**2.** The cosmetic composition according to claim 1, wherein said Genus cistanche is Cistanche tubulosa (Schenk.) Wight.

**3.** The cosmetic composition according to claim 1, wherein said extract of said Genus cistanche is Cistanche tubulosa (Schenk.) Wight which is extracted from the dry stems of Cistanche tubulosa (Schenk.) Wight.

**4.** The cosmetic composition according to claim 1, wherein said extract of said Cistanche tubulosa (Schenk.) Wight comprises at least 2% of acteoside by weight, and at least 4% of echinaoside by weight, based on the total weight of the extract.

**5.** The cosmetic composition according to claim 4, wherein said extract of said Cistanche tubulosa (Schenk.) Wight comprises 2-80% of acteoside by weight, and 4-80% of echinaoside by weight, based on the total weight of the extract.

**6.** The cosmetic composition according to claim 1, wherein said cosmetic composition is in a form of facial mask, lotion, facial cream, ointment or cleaning solution.

**7.** A process for preparing the cosmetic composition of any one of claims 1-6 , wherein said cosmetic composition containing an extract from Cistanche tubulosa (Schenk.) Wight, comprising the steps of:
a. grinding the dry stem of cistanche tubulosa (Schenk.) Wight to form a dry powder;
b. Soaking said dry powder of cistanche tubulosa (Schenk.) Wight with water or alcohol and refluxing the mixture at a temperature of 50-100°C;
c. Concentrating the supernatant obtained in step b under a decreased pressure and centrifuging the concentrate thus obtained;
d. adding the centrifugate obtained in step c onto a column which is packed with a macro-porous resin such as SEPABEADA;
e. eluting said column with 10-60% of alcohol or deionized water;
f. Collecting the eluant and subjecting it to be dried under vacuum, crushed and sieved to obtain an extract containing phenylethanoid glycosides.

**9.** The process for preparing any form of cosmetic composition containing an extract from Cistanche tubulosa (Schenk.) Wight according to claim 6, wherein said refluxing in step b is repeated 2-4 times and the temperation is from 70 to 90°C.

**10.** Use of any cosmetic composition of claims 1-5 for the freckle-removing in skin.

**11.** Use of any cosmetic composition of claims 1-5 for the whitening in skin.

**12.** Use of any cosmetic composition of claims 1-5 for improving the appearance of skin to be youthful.
